# EUROPÄISCHE PATENTSCHRIFT

(11) **EP 2 399 569 B1**
(45) Veröffentlichungstag und Bekanntmachung des Hinweises auf die Patenterteilung: **18.07.2018**
(21) Anmeldenummer: 10195194.5
(22) Anmeldetag: 15.12.2010
(51) Int. Cl.: A61K 8/49, A61Q 5/06, C09B 44/04, C09B 44/10

(54) **Kationische Azofarbstoffe mit einer speziellen Gruppierung des 4-Aza-1-azoniabicyclo[2.2.2]octans und Mittel zum Färben von keratinhaltigen Fasern**
Cationic azo dyes with a special grouping of the 4-Aza-1-azoniabicyclo[2.2.2]octane and means of colouring keratinic fibres
Colorants azoïques cationiques dotés d'un groupage spécifique du 4-Aza-1-azoniabicyclo[2.2.2]octane et moyen de coloration de fibres kératiniques

(30) Priorität: 23.06.2010 DE 102010030439
(43) Veröffentlichungstag der Anmeldung: 28.12.2011
(73) Patentinhaber: Henkel AG & Co. KGaA, 40589 Düsseldorf (DE)
(72) Erfinder: Groß, Wibke, 41836, Hückelhoven (DE); Giesa, Helmut, 40670, Meerbusch (DE); Kroos, Astrid, 40789, Monheim (DE)

(56) Entgegenhaltungen:
- EP-A1- 1 820 537
- CH-A- 552 032
- John F. Corbett: "Hair Dyes" In: "The Chemistry of Synthetic Dyes", 1 January 1971 (1971-01-01), XP55329462, vol. V, pages 475-478,

## Beschreibung

Die Erfindung betrifft neue kationische Farbstoffe sowie Mittel zum Färben und gegebenenfalls gleichzeitigen Aufhellen von keratinhaltigen Fasern, insbesondere menschlichen Haaren, die diese Farbstoffe enthalten, die Verwendung dieser kationischen Farbstoffe zum Färben von Haaren sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

CH 0552032 beschreibt Verfahren zur Herstellung von basischen Farbstoffen, die zum Färben von Polyesterfasern und Wolle geeignet sind. Im Beispielteil wird ein kationischer Farbstoff offenbart, der eine Gruppierung des 4-Aza-1-azoniabicyclo[2.2.2]octans trägt.

EP 1820537 A1 betrifft neue kationische Farbstoffe, die die Haare intensiv färben und oxidationsstabil sein sollen. Kennzeichnend für die in EP 1820537 A1 offenbarten Farbstoffe ist die Anwesenheit einer kationischen Gruppe Q, bei der es sich um eine aromatische oder nichtaromatische, substituierte oder unsubstituierte 4-6-gliedrige heterozyklische Ammoniumgruppe handelt.

Aufgabe der vorliegenden Erfindung ist es, neuartige direktziehende Farbstoffe bereit zu stellen, die sich ausgezeichnet für einen Einsatz in der Haarfärbung eignen und durch gute Echtheitseigenschaften, wie beispielsweise Licht-, Reib- und Waschechtheit sowie Schweiß- und Kaltwellechtheit auszeichnen. Im Falle der gleichzeitigen Anwendung mit Oxidationsfarbstoffen und/oder Oxidationsmitteln sollen die Direktzieher eine ausreichende Stabilität gegen H₂O₂ besitzen und ihre positiven Echtheits- und Färbeeigenschaften nicht verlieren. Darüber hinaus dürfen die Färbemittel kein oder lediglich ein sehr geringes Sensibilisierungspotential aufweisen und dürfen keinesfalls mutagen wirken. Zudem sollten möglichst leuchtende und intensive Färbungen erzielt werden.

Es wurde überraschenderweise gefunden, dass Verbindungen der allgemeinen Formel (I) sich sehr gut als Direktzieher für die Haarfärbung eignen. In Ausfärbungen werden intensive orange, rote, violette und blaue Farbnuancen mit guten Echtheitseigenschaften erhalten. Die Direktzieher liefern ebenfalls bei gleichzeitiger Anwendung von Oxidationsmitteln wie Wasserstoffperoxid oder einem Gemisch aus Wasserstoffperoxid und Peroxidisulfaten leuchtende Nuancen ohne Abschwächung von Farbintensität und Farbbrillanz. Auf diese Weise wird das gleichzeitige Aufhellen und Färben von Haaren möglich, wodurch auch auf dunklem Haar eine leuchtende Farbgebung erzielt werden kann.

Färbemittel, enthaltend Verbindungen gemäß nachstehender Formel (I) sowie einige der entsprechenden Verbindungen selbst sind bislang nicht bekannt.

Gegenstand der vorliegenden Erfindung sind in einer ersten Ausführungsform Mittel zum Färben und gegebenenfalls gleichzeitigen Aufhellen von menschlichen Haaren, enthaltend mindestens eine kationische Verbindung der Formel (I): 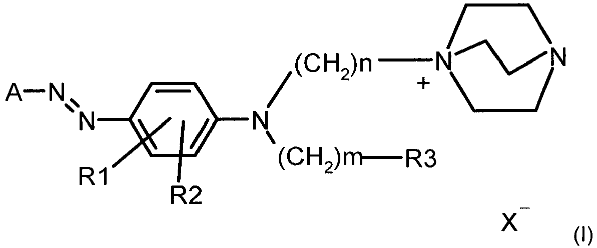 in der
- A: für einen gegebenenfalls substituierten Benzenring oder einen gegebenenfalls substituierten Naphthalenring der allgemeinen Formeln (II) oder (III) steht: 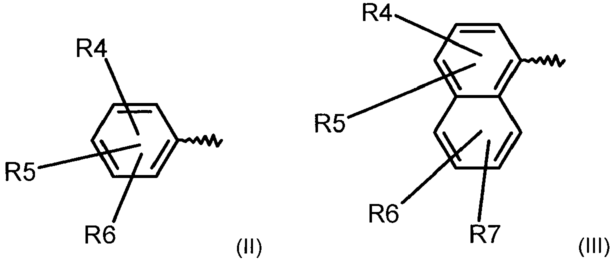 in denen
R⁴, R⁵, R⁶, R⁷ unabhängig voneinander für ein Wasserstoffatom, eine Nitrogruppe, eine Sulfonsäuregruppe, eine Nitrilgruppe, ein Halogenatom (Chlorid, Bromid oder Fluorid), eine Carbamoylgruppe, eine C₁-C₆-Alkylsulfonylgruppe, eine Sulfamoylgruppe oder eine C₁-C₆-Alkoxygruppe stehen, oder
- A: für einen der nachfolgend aufgeführten Heterocyclen (IV) bis (XV) steht 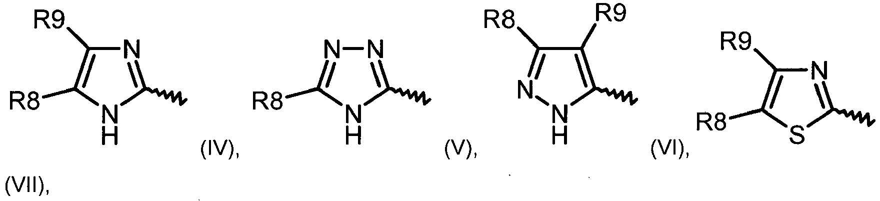 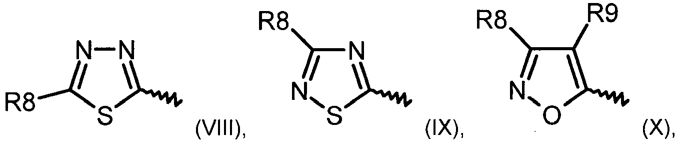 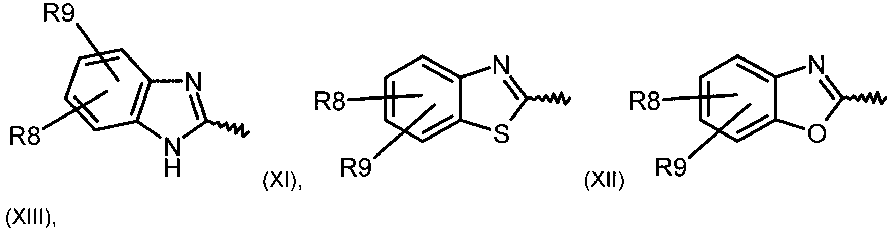 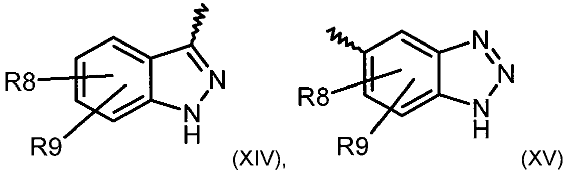
in denen
- R⁸, R⁹: unabhängig voneinander für ein Wasserstoffatom, eine Nitrogruppe, eine Nitrilgruppe, ein Halogenatom, eine C₁-C₆-Alkylsulfonylgruppe, eine Carbamoylgruppe, eine C₁-C₆-Alkylgruppe, eine C₁-C₆-Alkoxygruppe oder eine Phenylgruppe stehen,
- R¹, R², R³: stehen unabhängig voneinander für ein Wasserstoffatom, eine C₁-C₆-Alkylgruppe, eine C₂-C₆-Alkenylgruppe, eine C₁-C₆-Alkoxygruppe, ein Halogenatom (Cl, Br, J oder F), eine Hydroxygruppe, eine Aminogruppe, eine Carboxylgruppe, eine C₁-C₆-Alkylaminogruppe, eine C₁-C₆-Dialkylaminogruppe, eine Nitrogruppe, eine Nitrilgruppe, eine Hydroxy-C₁-C₆-alkyloxygruppe oder eine Ayclaminogruppe,
- n, m: stehen unabhängig voneinander für eine ganze Zahl von 1 - 6,
- X⁻: steht für ein einwertiges Anion, vorzugsweise für Halogenid, Hydrogensulfat, ½ Sulfat, p-Benzolsulfonat, p-Toluolsulfonat, Acetat, Tetrafluoroborat, Trifluormethansulfonat, Hexafluorophosphat, Methansulfat oder Methansulfonat.

Unter keratinhaltigen Fasern sind menschliche Haare zu verstehen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise Blondierungen.

Beispiele für (C₁ bis C₆)-Alkylreste sind die Gruppen Methyl, Ethyl, Propyl, Isopropyl, n-Butyl, sec-Butyl und tert.-Butyl, n-Pentyl und n-Hexyl. Propyl, Ethyl und Methyl sind bevorzugte Alkylreste. Beispiele für entsprechende cyclische Alkylgruppen sind Cyclopentyl und Cyclohexyl.

Beispiele für bevorzugte (C₂ bis C₆)-Alkenylreste sind Vinyl und Allyl.

Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyethylgruppe genannt werden. Eine 2-Hydroxyethylgruppe ist besonders bevorzugt.

Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe.

Erfindungsgemäß bevorzugte (C₁ bis C₆)-Alkoxygruppen sind beispielsweise eine Methoxy- oder eine Ethoxygruppe.

Die Methoxyethyl-, Ethoxyethyl-, Methoxypropyl-, Methoxybutyl-, Ethoxybutyl- und die Methoxyhexylgruppe sind Beispiele für erfindungsgemäße (C₁ bis C₆)-Alkoxy-(C₂ bis C₆)-alkylgruppen.

Eine bevorzugte Hydroxy-(C₁-C₆)-alkoxygruppe ist die 2-Hydroxyethoxygruppe.

Beispiele für Halogenatome sind F-, Cl-, Br- oder I-Atome, wobei Br- oder Cl-Atome ganz besonders bevorzugt sind.

Beispiele für (C₁ bis C₆)-Alkylaminogruppen sind Methylamino-, Ethylamino- und Propylaminiogruppen.

Beispiele für (C₁ bis C₆)-Dialkylaminogruppen sind Dimethylamino-, Diethylamino- und Dipropylaminiogruppen.

Bevorzugte Aryl-(C₁ bis C₆)-alkylgruppen sind Benzyl und 2-Phenylethyl.

Die zufriedenstellende Erfüllung der Aufgabenstellung ist insbesondere dann möglich, wenn die Reste R1 und R2 unabhängig voneinander für ein Wasserstoffatom, eine C₁-C₆-Alkylgruppe, eine C₁-C₆-Alkoxygruppe oder ein Halogenatom (Cl, Br, J oder F) stehen.

Wenn R3 für ein Wasserstoffatom, eine C₁-C₆-Alkylgruppe, eine C₁-C₆-Alkoxygruppe, ein Halogenatom, eine Hydroxygruppe oder eine Nitrilgruppe steht, so ist dies ebenfalls bevorzugt. n und m stehen vorteilhafterweise unabhängig voneinander für die Zahlen 2 oder 3, insbesondere für die Zahl 2.

In einer ersten Ausführungsform steht A für einen gegebenenfalls substituierten Benzenring oder einen gegebenenfalls substituierten Naphthalenring der allgemeinen Formeln (II) oder (III). Die allgemeine Formel (II) ist hierbei besonders bevorzugt.

Erfindungsgemäße Mittel zum Färben und gegebenenfalls gleichzeitigen Aufhellen keratinischer Fasern enthalten Verbindungen der Formel (I), in der die Gruppierung A für einen gegebenenfalls substituierten Benzenring oder einen gegebenenfalls substituierten Naphthalenring der allgemeinen Formeln (II) oder (III) steht: 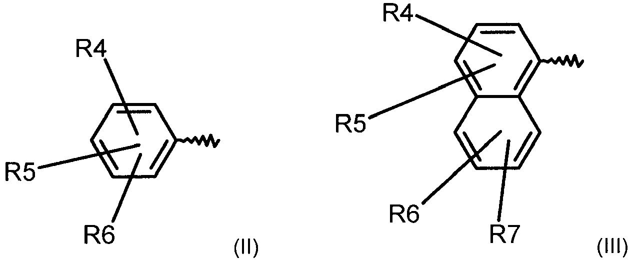 in denen
- R⁴, R⁵, R⁶, R⁷: unabhängig voneinander für ein Wasserstoffatom, eine Nitrogruppe, eine Sulfonsäuregruppe, eine Nitrilgruppe, ein Halogenatom (Chlorid, Bromid oder Fluorid), eine Carbamoylgruppe, eine C₁-C₆-Alkylsulfonylgruppe, eine Sulfamoylgruppe oder eine C₁-C₆-Alkoxygruppe stehen.

Insbesondere bevorzugte erfindungsgemäße Mittel zum Färben und gegebenenfalls gleichzeitigen Aufhellen keratinischer Fasern enthalten mindestens eine kationische Verbindung aus der Gruppe der
- Salze des 1-[2-({4-[2-(4-Nitrophenyl)diazenyl]phenyl}ethylamino)ethyl]-4-aza-1-azoniabicyclo[2.2.2]octans
- Salze des 1-[2-({4-[2-(4-Nitrophenyl)diazenyl]-3-methylphenyl}ethylamino)ethyl]-4-aza-1-azoniabicyclo[2.2.2]octans
- Salze des 1-[2-({4-[2-(4-Nitrophenyl)diazenyl]-3-chlorphenyl}ethylamino)ethyl]-4-aza-1-azoniabicyclo[2.2.2]octans
- Salze des 1-[2-({4-[2-(2-Cyano-4-nitrophenyl)diazenyl]phenyl}ethylamino)ethyl]-4-aza-1-azoniabicyclo[2.2.2]octans
- Salze des 1-[2-({4-[2-(2-Cyano-4-nitrophenyl)diazenyl]-3-methylphenyl}ethylamino)ethyl]-4-aza-1-azoniabicyclo[2.2.2]octans
- Salze des 1-[2-({4-[2-(2-Cyano-4-nitrophenyl)diazenyl]-3-chlorphenyl}ethylamino)ethyl]-4-aza-1-azoniabicyclo[2.2.2]octans
- Salze des 1-[2-({4-[2-(2-Chlor-4-nitrophenyl)diazenyl]phenyl}ethylamino)ethyl]-4-aza-1-azoniabicyclo[2.2.2]octans
- Salze des 1-[2-({4-[2-(2-Chlor-4-nitrophenyl)diazenyl]-3-methylphenyl}ethylamino)ethyl]-4-aza-1-azoniabicyclo[2.2.2]octans
- Salze des 1-[2-({4-[2-(2-Chlor-4-nitrophenyl)diazenyl]-3-chlorphenyl}ethylamino)ethyl]-4-aza-1-azoniabicyclo[2.2.2]octans
- Salze des 1-[2-({4-[2-(2-Chlor-6-cyano-4-nitrophenyl)-diazenyl]phenyl}ethylamino)ethyl]-4-aza-1-azoniabicyclo[2.2.2]octans
- Salze des 1-[2-({4-[2-(2-Chlor-6-cyano-4-nitrophenyl)diazenyl]-3-methylphenyl}ethylamino)ethyl]-4-aza-1-azoniabicyclo[2.2.2]octans
- Salze des 1-[2-({4-[2-(2-Chlor-6-cyano-4-nitrophenyl)diazenyl]-3-chlorphenyl}ethylamino)ethyl]-4-aza-1-azoniabicyclo[2.2.2]octans
- Salze des 1-[2-({4-[2-(2,6-Dichlor-4-nitrophenyl)-diazenyl]phenyl}ethylamino)ethyl]-4-aza-1-azoniabicyclo[2.2.2]octans
- Salze des 1-[2-({4-[2-(2,6-Dichlor-4-nitrophenyl)diazenyl]-3-methylphenyl}ethylamino)ethyl]-4-aza-1-azoniabicyclo[2.2.2]octans
- Salze des 1-[2-({4-[2-(2,6-Dichlor-4-nitrophenyl)diazenyl]-3-chlorphenyl}ethylamino)ethyl]-4-aza-1-azoniabicyclo[2.2.2]octans
- Salze des 1-[2-({4-[2-(4-Methoxy-2-nitrophenyl)diazenyl]phenyl}ethylamino)ethyl]-4-aza-1-azoniabicyclo[2.2.2]octans
- Salze des 1-[2-({4-[2-(4-Methoxy-2-nitrophenyl)diazenyl]-3-methylphenyl}ethylamino)ethyl]-4-aza-1-azoniabicyclo[2.2.2]octans
- Salze des 1-[2-({4-[2-(4-Methoxy-2-nitrophenyl)diazenyl]-3-chlorphenyl}ethylamino)ethyl]-4-aza-1-azoniabicyclo[2.2.2]octans
- Salze des 1-[2-({4-[2-(2-Methoxy-4-nitrophenyl)diazenyl]phenyl}ethylamino)ethyl]-4-aza-1-azoniabicyclo[2.2.2]octans
- Salze des 1-[2-({4-[2-(2-Methoxy-4-nitrophenyl)diazenyl]-3-methylphenyl}ethylamino)ethyl]-4-aza-1-azoniabicyclo[2.2.2]octans
- Salze des 1-[2-({4-[2-(2-Methoxy-4-nitrophenyl)diazenyl]-3-chlorphenyl}ethylamino)ethyl]-4-aza-1-azoniabicyclo[2.2.2]octans
- Salze des 1-[2-({4-[2-(2-Methyl-4-nitrophenyl)diazenyl]phenyl}ethylamino)ethyl]-4-aza-1-azoniabicyclo[2.2.2]octans
- Salze des 1-[2-({4-[2-(2-Methyl-4-nitrophenyl)diazenyl]-3-methylphenyl}ethylamino)ethyl]-4-aza-1-azoniabicyclo[2.2.2]octans
- Salze des 1-[2-({4-[2-(2-Methyl-4-nitrophenyl)diazenyl]-3-chlorphenyl}ethylamino)ethyl]-4-aza-1-azoniabicyclo[2.2.2]octans

Besonders bevorzugte Verbindungen dieser Ausführungsform sind
- 1-[2-({4-[2-(2-Cyano-4-nitrophenyl)diazenyl]phenyl}ethylamino)ethyl]-4-aza-1-azoniabicyclo[2.2.2]octan, Chlorid
- 1-[2-({4-[2-(2-Cyano-4-nitrophenyl)diazenyl]phenyl}ethylamino)ethyl]-4-aza-1-azoniabicyclo[2.2.2]octan, Bromid
- 1-[2-({4-[2-(2-Cyano-4-nitrophenyl)diazenyl]phenyl}ethylamino)ethyl]-4-aza-1-azoniabicyclo[2.2.2]octan, Hydrogensulfat
- 1-[2-({4-[2-(2-Cyano-4-nitrophenyl)diazenyl]phenyl}ethylamino)ethyl]-4-aza-1-azoniabicyclo[2.2.2]octan, Benzolsulfonat
- 1-[2-({4-[2-(2-Cyano-4-nitrophenyl)diazenyl]phenyl}ethylamino)ethyl]-4-aza-1-azoniabicyclo[2.2.2]octan, p-Toluolsulfonat

Weitere besonders bevorzugte Salze sind weiter unten bei der Offenbarung der Verbindungen an sich explizit aufgeführt. Erfindungsgemäße Mittel, die die dort offenbarten Salze enthalten, sind ebenfalls besonders bevorzugt.
Im Rahmen einer zweiten Ausführungsform hat es sich zur Lösung der erfindungsgemäßen Aufgabenstellung ebenfalls als besonders geeignet erwiesen, wenn die Gruppierung A für einen der nachfolgenden Heterocyclen (IV) bis (XV) steht.
Erfindungsgemäße Mittel zum Färben und gegebenenfalls gleichzeitigen Aufhellen keratinischer Fasern enthalten Verbindungen der Formel (I), in der die Gruppierung A für einen der nachfolgend aufgeführten Heterocyclen (IV) bis (XV) steht 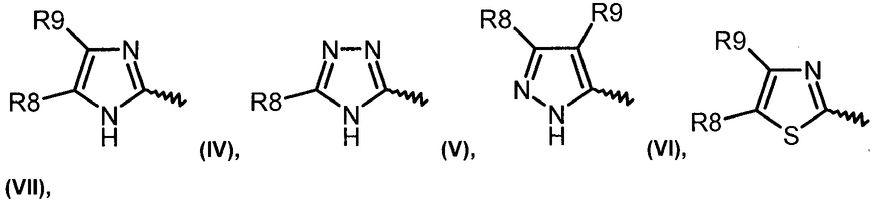 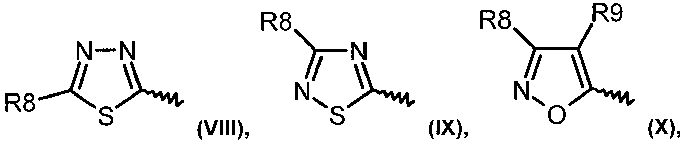 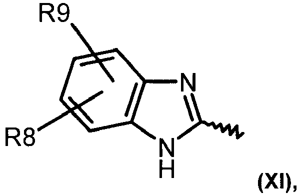 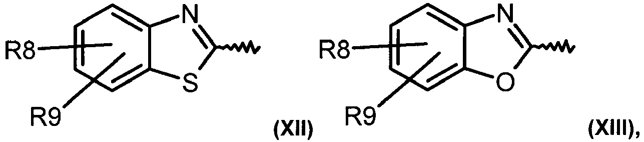 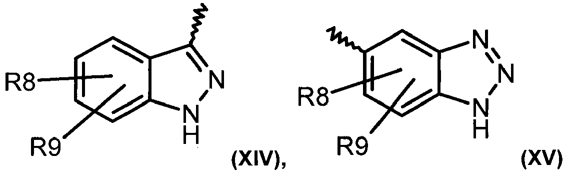 in denen
- R⁸, R⁹: unabhängig voneinander für ein Wasserstoffatom, eine Nitrogruppe, eine Nitrilgruppe, ein Halogenatom, eine C₁-C₆-Alkylsulfonylgruppe, eine Carbamoylgruppe, eine C₁-C₆-Alkylgruppe, eine C₁-C₆-Alkoxygruppe oder eine Phenylgruppe stehen.

Insbesondere bevorzugte erfindungsgemäße Mittel zum Färben und gegebenenfalls gleichzeitigen Aufhellen keratinischer Fasern enthalten mindestens eine kationische Verbindung aus der Gruppe der
- Salze des 1-[2-({4-[2-(4,5-Dicyanoimidazol-2-yl)diazenyl]phenyl}ethylamino)ethyl]-4-aza-1-azoniabicyclo[2.2.2]octans
- Salze des 1-[2-({4-[2-(4,5-Dicyanoimidazol-2-yl)diazenyl]-3-methylphenyl}ethylamino)ethyl]-4-aza-1-azoniabicyclo[2.2.2]octans
- Salze des 1-[2-({4-[2-(4,5-Dicyanoimidazol-2-yl)diazenyl]-3-chlorphenyl}ethylamino)ethyl]-4-aza-1-azoniabicyclo[2.2.2]octans
- Salze des 1-[2-({4-[2-(1,2,4-4H-Triazol-3-yl)diazenyl]phenyl}ethylamino)ethyl]-4-aza-1-azoniabicyclo[2.2.2]octans
- Salze des 1-[2-({4-[2-(1,2,4-4H-Triazol-3-yl)diazenyl]-3-methylphenyl}ethylamino)ethyl]-4-aza-1-azoniabicyclo[2.2.2]octans
- Salze des 1-[2-({4-[2-(1,2,4-4H-Triazol-3-yl)diazenyl]-3-chlorphenyl}ethylamino)ethyl]-4-aza-1-azoniabicyclo-[2.2.2]octans
- Salze des 1-[2-({4-[2-(5-Phenyl-1H-pyrazol-3-yl)diazenyl]phenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-methylphenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-chlorphenyl}ethylamino)ethyl]-4-aza-1-azoniabicyclo-[2.2.2]octans
- Salze des 1-[2-({4-[2-(Thiazol-2-yl)diazenyl]phenyl}ethylamino)ethyl]-4-aza-1-azoniabicyclo[2.2.2]octans
- Salze des 1-[2-({4-[2-(Thiazol-2-yl)diazenyl]-3-methylphenyl}ethylamino)ethyl]-4-aza-1-azoniabicyclo[2.2.2]octans
- Salze des 1-[2-({4-[2-(Thiazol-2-yl)diazenyl]-3-chlorphenyl}ethylamino)ethyl]-4-aza-1-azoniabicyclo-[2.2.2]octans
- Salze des 1-[2-({4-[2-(5-Nitrothiazol-2-yl)diazenyl]phenyl}ethylamino)ethyl]-4-aza-1-azoniabicyclo[2.2.2]octans
- Salze des 1-[2-({4-[2-(5-Nitrothiazol-2-yl)diazenyl]-3-methylphenyl}ethylamino)ethyl]-4-aza-1-azoniabicyclo[2.2.2]octans
- Salze des 1-[2-({4-[2-(5-Nitrothiazol-2-yl)diazenyl]-3-chlorphenyl}ethylamino)ethyl]-4-aza-1-azoniabicyclo-[2.2.2]octans
- Salze des 1-[2-({4-[2-(3-Phenyl-1,2,4-thiadiazol-5-yl)diazenyl]phenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-methylphenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-chlorphenyl}ethylamino)ethyl]-4-aza-1-azoniabicyclo-[2.2.2]octans
- Salze des 1-[2-({4-[2-(5-Nitro-1H-benzimidazol-2-yl)diazenyl]phenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-methylphenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-chlorphenyl}ethylamino)ethyl]-4-aza-1-azoniabicyclo-[2.2.2]octans
- Salze des 1-[2-({4-[2-(5-Nitro-benzothiazol-2-yl)diazenyl]phenyl}ethylamino)ethyl]-4-aza-1-azoniabicyclo[2.2.2]octans
- Salze des 1-[2-({4-[2-(5-Nitro-benzothiazol-2-yl)diazenyl]-3-methylphenyl}ethylamino)ethyl]-4-aza-1-azoniabicyclo[2.2.2]octans
- Salze des 1-[2-({4-[2-(5-Nitro-benzothiazol-2-yl)diazenyl]-3-chlorphenyl}ethylamino)ethyl]-4-aza-1-azoniabicyclo-[2.2.2]octans
- Salze des 1-{2-[(4-{2-[6-(Methylsulfonyl)benzothiazol-2-yl]diazeny}phenyl)-ethylamino]ethyl}-4-aza-1-azoniabicyclo[2.2.2]octans
- Salze des 1-{2-[(4-{2-[6-(Methylsulfonyl)benzothiazol-2-yl]diazeny}-3-methylphenyl)-ethylamino]ethyl}-4-aza-1-azoniabicyclo[2.2.2]octans
- Salze des 1-{2-[(4-{2-[6-(Methylsulfonyl)benzothiazol-2-yl]diazeny}-3-chlorphenyl)-ethylamino]ethyl}-4-aza-1-azoniabicyclo[2.2.2]octans
- Salze des 1-[2-({4-[2-(5-Methoxybenzothiazol-2-yl)diazenyl]phenyl}ethylamino)ethyl]-4-aza-1-azoniabicyclo[2.2.2]octans
- Salze des 1-[2-({4-[2-(5-Methoxybenzothiazol-2-yl)diazenyl]-3-methylphenyl}ethylamino)ethyl]-4-aza-1-azoniabicyclo[2.2.2]octans
- Salze des 1-[2-({4-[2-(5-Methoxybenzothiazol-2-yl)diazenyl]-3-chlorphenyl}ethylamino)ethyl]-4-aza-1-azoniabicyclo-[2.2.2]octans
- Salze des 1-[2-({4-[2-(1H-Benzotriazol-6-yl)diazenyl]phenyl}ethylamino)ethyl]-4-aza-1-azoniabicyclo[2.2.2]octans
- Salze des 1-[2-({4-[2-(1H-Benzotriazol-6-yl)diazenyl]-3-methylphenyl}ethylamino)ethyl]-4-aza-1-azoniabicyclo[2.2.2]octans
- Salze des 1-[2-({4-[2-(1H-Benzotriazol-6-yl)diazenyl]-3-chlorphenyl}ethylamino)ethyl]-4-aza-1-azoniabicyclo[2.2.2]octans

Besonders bevorzugte Verbindungen dieser zweiten Ausführungsform sind
- 1-[2-({4-[2-(5-Nitrothiazol-2-yl)diazenyl]phenyl}ethylamino)ethyl]-4-aza-1-azoniabicyclo[2.2.2]octan, Chlorid
- 1-[2-({4-[2-(5-Nitrothiazol-2-yl)diazenyl]phenyl}ethylamino)ethyl]-4-aza-1-azoniabicyclo[2.2.2]octan, Bromid
- 1-[2-({4-[2-(5-Nitrothiazol-2-yl)diazenyl]phenyl}ethylamino)ethyl]-4-aza-1-azoniabicyclo[2.2.2]octan, Hydrogensulfat
- 1-[2-({4-[2-(5-Nitrothiazol-2-yl)diazenyl]phenyl}ethylamino)ethyl]-4-aza-1-azoniabicyclo[2.2.2]octan, Benzolsulfonat
- 1-[2-({4-[2-(5-Nitrothiazol-2-yl)diazenyl]phenyl}ethylamino)ethyl]-4-aza-1-azoniabicyclo[2.2.2]octan, p-Toluolsulfonat

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 1 ppm und unterhalb von 10 Gew.-%, jeweils bezogen auf das gesamte Mittel. Bevorzugte erfindungsgemäße Mittel zum Färben und gegebenenfalls gleichzeitigen Aufhellen keratinischer Fasern sind dadurch gekennzeichnet, daß sie die Verbindung(en) der Formel (I) in Mengen von 0,001 bis 5 Gew.-%, vorzugsweise von 0,0025 bis 2,5 Gew.-%, besonders bevorzugt von 0,005 bis 1,5 Gew.-% und insbesondere von 0,01 bis 1 Gew.-%, jeweils bezogen auf das gesamte Mittel, enthalten.
Die erfindungsgemäßen Mittel dienen der Farbveränderung menschlicher Haare. Die Farbveränderung kann allein aufgrund der kationischen Verbindung(en) der Formel (I) erfolgen, die erfindungsgemäßen Mittel können aber auch zusätzlich weitere farbverändernde Substanzen enthalten, beispielsweise direktziehende Farbstoffe und/oder Oxidationsfärbemittel.

Erfindungsgemäße Mittel zum Färben und gegebenenfalls gleichzeitigen Aufhellen keratinischer Fasern, die zusätzlich - bezogen auf ihr Gewicht - 0,001 bis 5 Gew.-% eines oder mehrerer Oxidationsfarbstoffvorprodukte und/oder direktziehender Farbstoffe enthalten, sind dabei bevorzugt. Für permanente, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Diese weiteren farbgebenden Substanzen werden nachstehend beschrieben: Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.
Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation. Wenn die zyklische Verbindung ein Sechsring ist, so befinden sich die besagten Gruppen bevorzugt in ortho-Position oder meta-Position zueinander.
Für temporäre Färbungen werden üblicherweise Färbe- oder Tönungsmittel verwendet, die als färbende Komponente so genannte Direktzieher enthalten. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.
Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.
Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Eine weitere Möglichkeit zur Farbveränderung bietet die Verwendung von Färbemitteln, welche sogenannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in einem nichtoxidativen Prozess der so genannten Oxofärbung Farbstoffe aus. Die resultierenden Färbungen besitzen teilweise Farbechtheiten auf der keratinhaltigen Faser, die mit denen der Oxidationsfärbung vergleichbar sind.
Das mit der schonenden Oxofärbung erzielbare Nuancenspektrum ist sehr breit und die erhaltene Färbung weist oftmals eine akzeptable Brillanz und Farbtiefe auf. Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.
Als Oxofarbstoffvorprodukte kommt bevorzugt eine Kombination aus
- mindestens einer Verbindung, die mindestens eine reaktive Carbonylgruppe enthält (Komponente (Oxo 1))
   mit mindestens einer Verbindung (Komponente Oxo2)
- Verbindungen, ausgewählt aus
   (Oxo2a) CH-aciden Verbindungen
   und/oder aus
   (Oxo2b) Verbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterozyklischen Verbindungen und aromatischen Hydroxyverbindungen
   zum Einsatz.

Die erfindungsgemäßen Mittel enthalten mit besonderem Vorzug zusätzlich Wasserstoffperoxid. Hier sind erfindungsgemäße Mittel zum Färben und gegebenenfalls gleichzeitigen Aufhellen keratinischer Fasern besonders bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Das Wasserstoffperoxid kann auch in Form von dessen Anlagerungsverbindungen an feste Träger eingesetzt werden, bevorzugt wird Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, eingesetzt. Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Für eine Farbveränderung mittels Aufhellung bzw. Bleichung des Substrats, beispielsweise der Haare, wird bevorzugt in den erfindungsgemäßen kosmetischen Mitteln neben den Oxidationsmitteln zusätzlich mindestens ein Bleichverstärker eingesetzt.

Bleichverstärker werden bevorzugt zur Steigerung der Bleichwirkung des Oxidationsmittels, insbesondere des Wasserstoffperoxids, eingesetzt. Geeignete Bleichverstärker sind
(BV-i) Verbindungen, die unter Perhydrolysebedingungen aliphatische Peroxocarbonsäuren und/oder gegebenenfalls substituierte Perbenzoesäure ergeben, und/oder
(BV-ii) Carbonatsalze und/oder Hydrogencarbonatsalze, und/oder
(BV-iii) organische Carbonate, und/oder
(BV-iv) Carbonsäuren, und/oder
(BV-v) Peroxoverbindungen.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die sauerstoff- und/oder stichstoffgebundene Acylgruppen mit der genannten Anzahl an Kohlenstoffatomen und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Die Carbonat- bzw. Hydrogencarbonatsalze werden bevorzugt ausgewählt aus mindestens einer Verbindung der Gruppe bestehend aus Ammonium-, Alkali- (insbesondere Natrium- und Kalium-), sowie Erdalkali- (insbesondere Calcium-), -carbonatsalzen und -hydrogencarbonatsalzen.

Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Bevorzugt nutzbare organische Carbonate werden ausgewählt aus mindestens einer Verbindung der Gruppe der Kohlensäuremonoester und/oder aus mindestens einer Verbindung der Gruppe der Kohlensäuremonoamide.

Bevorzugt verwendbare Kohlensäuremonoester sind die Kohlensäuremonoester der Formel (BV-1), 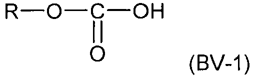 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (BV-1) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁₋C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (BV-1) ausgewählt ist aus Methyl-, Ethyl-, n-Propyl-, isoPropyl-, n-Butyl-, iso-Butyl-, *tert*-Butyl- sowie Hydroxymethyl- und Hydroxyethyl-Resten. Bevorzugte Kohlensäuremonoamide sind die Verbindungen der Formel (BV-2), 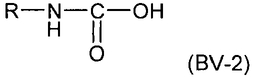 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht. In Formel (BV-2) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (BV-2) sind dadurch gekennzeichnet, daß der Rest R in Formel (BV-2) ausgewählt ist aus Methyl-, Ethyl-, n-Propyl-, isoPropyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als bleichverstärkende Carbonsäure kann in den erfindungsgemäßen Zusammensetzungen bevorzugt mindestens eine Verbindung aus der Gruppe, bestehend aus Essigsäure, Milchsäure, Weinsäure, Zitronensäure, Salicylsäure und ortho-Phthalsäure, enthalten sein.

Bleichverstärker sind bevorzugt Peroxoverbindungen, insbesondere anorganische Peroxoverbindungen. Unter die erfindungsgemäß bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, Peroxidiphosphatsalze (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die Peroxidisulfate, insbesondere Ammoniumperoxidisulfat, bevorzugt. Hier sind erfindungsgemäße Mittel zum Färben und gegebenenfalls gleichzeitigen Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, -peroxomonosulfaten und - peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, jeweils bezogen auf das Gewicht des anwendungsbereiten Mittels, enthalten.

Weiterhin hat es sich als vorteilhaft erwiesen, wenn die erfindungsgemäßen Färbe- und/oder Aufhellmittel nichtionogene grenzflächenaktive Stoffe enthalten.

Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen. Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxy-stearat (Handelsprodukt: Dehymuls®PGPH (Henkel)).
- Sorbitan-Fettsäureester und alkoxylierte Sorbitan- Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO. Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Färbe- und/oder Aufhellmittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein. Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor. Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie das unter der Bezeichnung Dehyquart®F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Panthenol, Pantothensäure, Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Auswahl dieser weiteren Stoffe wird der Fachmann gemäß der gewünschten Eigenschaften der Mittel treffen.

Die erfindungsgemäßen Mittel können die Inhaltsstoffe in einem geeigneten wäßrigen, alkoholischen oder wäßrig-alkoholischen Träger enthalten. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist. Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Ethanol, n-Propanol, Isoropanol, n-Butanol, Propylenglykol, n- Butylenglykol, Glycerin, Diethylenglykolmonoethylether, Diethylenglykolmono-n- butylether, Phenoxyethanol und Benzylalkohol sowie ihren Mischungen.

Der pH-Wert der erfindungsgemäßen Mittel kann durch geeignete Inhaltstoffe wie Acidifizierungsmittel oder Alkalisierungsmittel in einem weiten Bereich eingestellt werden.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel (la) und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Für die Anwendung der erfindungsgemäßen Mittel auf dem Haar werden die Färbe- und/oder Aufhellmittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Färbe- und/oder Aufhellmittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Mittel zum Färben und gegebenenfalls gleichzeitigen Aufhellen keratinischer Fasern enthalten zusätzlich Cu-, Fe-, Mn-, Co-, Ce-, V-, Ru-Ionen oder Komplexe dieser Ionen, wobei bevorzugte Mittel 0,0001 bis 2,5 Gew.-%, vorzugsweise 0,001 bis 1 Gew.-% mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Mangandioxid (MnO₂).

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-Atomen in der Regel unter Bildung von Chelat-Komplexen reagieren, sind erfindungsgemäß einsetzbar. Die Polymer-gebundenen Liganden der entstehenden MetallKomplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Komplexierende Gruppen (Liganden) üblicher komplexbildender Polymere sind Iminodiessigsäure-, Hydroxychinolin-, Thioharnstoff-, Guanidin-, Dithiocarbamat-, Hydroxamsäure-, Amidoxim-, Aminophosphorsäure-, (cycl.) Polyamino-, Mercapto-, 1,3-Dicarbonyl- und Kronenether-Reste mit z. T. sehr spezif. Aktivitäten gegenüber Ionen unterschiedlicher Metalle. Basispolymere vieler auch kommerziell bedeutender komplexbildender Polymere sind Polystyrol, Polyacrylate, Polyacrylnitrile, Polyvinylalkohole, Polyvinylpyridine und Polyethylenimine. Auch natürliche Polymere wie Cellulose, Stärke od. Chitin sind komplexbildende Polymere. Darüber hinaus können diese durch polymeranaloge Umwandlungen mit weiteren Ligand-Funktionalitäten versehen werden.

Besonders bevorzugt ist im Rahmen der vorliegenden Erfindung der Einsatz eines oder mehrerer Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine.

Im Rahmen der vorliegenden Erfindung können alle Komplexbildner des Standes der Technik eingesetzt werden. Diese können unterschiedlichen chemischen Gruppen angehören. Vorzugsweise werden einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure,
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)glycin, N-(1,2-Dicarboxy-2-hydroxyethyl)-glycin, N-(1,2-Dicarboxy-2-hydroxyethyl)asparaginsäure oder Nitrilotriessigsäure (NTA),
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate hiervon,
d) Aminophosphonsäuren wie Ethylendiamintetra(methylenphosphonsäure), Diethylen-triaminpenta(methylenphosphonsäure) oder Nitrilotri(methylenphosphonsäure),
e) Phosphonopolycarbonsäuren wie 2-Phosphonobutan-1,2,4-tricarbonsäure sowie
f) Cyclodextrine.

Als Polycarbonsäuren a) werden im Rahmen dieser Patentanmeldung Carbonsäuren -auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Ebenso sind als weitere bevorzugte Komplexbildner polymere Aminodicarbonsäuren, deren Salze oder deren Vorläufersubstanzen zu nennen. Besonders bevorzugt sind Polyasparaginsäuren bzw. deren Salze und Derivate, die neben Cobuilder-Eigenschaften auch eine bleichstabilisierende Wirkung aufweisen.

Weitere geeignete Komplexbildner sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und OctaNatriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Erfindungsgemäß besonders bevorzugte Mittel enthalten einen oder mehrere Stoffe aus der Gruppe
(a) Nitrilotriessigsäure (NTA),
(b) Diethylenetriaminpentaesigsäure (DTPA),
(c) Ethylendiamindibernsteinsäure (EDDS),
(d) Ethylenediamindiglutarsäure (EDGA),
(e) 2- Hydroxypropylendiamindibernsteinsäure (HPDS),
(f) Glycinamid-N,N'- dibernsteinsäure (GADS),
(g) Ethylendiamin-N-N'-diglutarsäure (EDDG),
(h) 2-Hydroxypropylendiamin-N-N'-dibernsteinsäure (HPDDS),
(i) Ethylendiamintetraessigsäure (EDTA),
(j) Ethylendicysteinsäure (EDC),
(k) Diaminoalkyldi(sulfobernsteinsäure) (DDS),
(l) Ethylendiamine-N-N'-bis(ortho-hydroxyphenylesigsäure (EDDHA),
(m) N-2-hydroxyethyl-N,N-diessigsäure,
(n) Glyceryliminodiessigsäure,
(o) Iminodiessigsäure-N-2-hydroxypropylsulfonsäure,
(p) Asparaginsäure-N-carboxymethyl-N-2,5-hydroxypropyl-3-sulfonsäure,
(q) 8-Alanin-N,N'-diessigsäure,
(r) Asparaginsäure-N,N'-diessigsäure,
(s) Asparaginsäure-N-monoessigsäure,
(t) Dipicolinsäure,
(u) sowie deren Salze und/oder Derivate

Aus den vorstehend genannten Stoffgruppen sind einige Vertreter im Rahmen der vorliegenden Erfindung besonders bevorzugt. Erfindungsgemäß besonders bevorzugte Mittel zum Färben und gegebenenfalls gleichzeitigen Aufhellen keratinischer Fasern enthalten zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine,
wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

Bevorzugte erfindungsgemäße Mittel werden wasserarm bzw. wasserfrei formuliert. Erfindungsgemäß besonders bevorzugte Mittel sind dadurch gekennzeichnet, daß sie weniger als 5 Gew.-%, vorzugsweise weniger als 2 Gew.-%, besonders bevorzugt weniger als 1 Gew.-% und insbesondere weniger als 0,5 Gew.-% Wasser enthalten, wobei bevorzugte Mittel wasserfrei sind. Der Wassergehalt der Mittel läßt sich beispielsweise mittels Titration nach Karl Fischer bestimmen.

Ein zweiter Gegenstand der vorliegenden Erfindung ist ein Verfahren zum Färben und gegebenenfalls gleichzeitigen Aufhellen von Keratinfasern, insbesondere menschlichen Haaren, bei dem
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein erfindungsgemäßes Mittel ist. Die erfindungsgemäßen Mittel können demnach als Einkomponentenmittel (Färbe- und Aufhellmittel M2 bzw. Nachbehandlungsmittel M4), als Zweikomponenten Mittel (M2 + M3) oder als Dreikomponentenmittel (M2 + M3 + M4) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponenten hergestellt.

Ein Färbe- und Aufhellungsverfahren, bei dem die Aufhellcreme und das Oxidationsmittel zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem erfindungsgemäßen Mittel zu einer homogenen Zusammensetzung vermischt und diese auf das Haar aufgebracht wird.

In bevorzugten erfindungsgemäßen Verfahren dieser Art enthält die Zusammensetzung auf wäßriger Grundlage bezogen auf ihr Gewicht 1 bis 20 Gew.-%, vorzugsweise 2 bis 10 Gew.-% und insbesondere 3 bis 6 Gew.-% Wasserstoffperoxid, berechnet als 100%iges H₂O₂. Weiter bevorzugte erfindungsgemäße Verfahren dieser Art sind dadurch gekennzeichnet, daß die Zusammensetzung auf wäßriger Grundlage, enthaltend Wasserstoffperoxid, mit einem erfindungsgemäßen Mittel im Gewichtsverhältnis 1:5 bis 10:1, vorzugsweise 1:2 bis 5:1 und insbesondere 1:2 bis 2:1 zu einer homogenen Zusammensetzung vermischt, und diese auf das Haar aufgebracht wird.

Alternativ kann - wie vorstehend erwähnt - auch ein Dreikomponentensystem zur Anwendung gelangen. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem erfindungsgemäßen Mittel zu einer homogenen Zusammensetzung vermischt, und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.
Besonders bevorzugt ist es, die Ausfärbung durch physikalische Maßnahmen zu unterstützen. Erfindungsgemäße Verfahren, bei denen die Anwendung durch Einwirkung von Wärme und/oder UV-Strahlung während der Einwirkzeit unterstützt wird, sind bevorzugte Ausführungsformen der vorliegenden Erfindung..
Die Verbindungen der allgemeinen Formel (I) eignen sich sehr gut als Direktzieher für die Haarfärbung. In Ausfärbungen werden extrem intensive Farbnuancen mit sehr guten
Echtheitseigenschaften, insbesondere im Rot- und Braunbereich erhalten. Die Direktzieher liefern ebenfalls bei gleichzeitiger Anwendung von Oxidationsmitteln wie Wasserstoffperoxid oder einem Gemisch aus Wasserstoffperoxid und Peroxidisulfaten leuchtende Nuancen ohne Abschwächung von Farbintensität und Farbbrillanz. Auf diese Weise wird das gleichzeitige Aufhellen und Färben von Haaren möglich, wodurch auch auf dunklem Haar eine leuchtende Farbgebung erzielt werden kann.

Ein weiterer Gegenstand der vorliegenden Erfindung ist daher die Verwendung von kationischen Verbindungen der Formel (I): 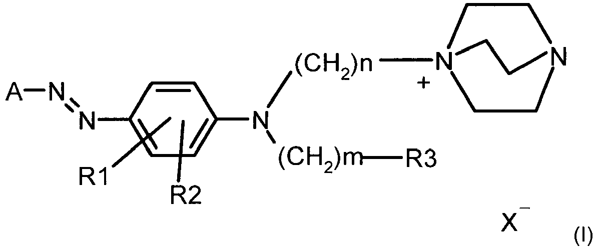 in der
- A: ein heterocyclisches oder carbocyclisches, gegebenenfalls substituiertes, ungeladenes Ringsystem ist,
- R¹, R², R³: stehen unabhängig voneinander für ein Wasserstoffatom, eine C₁-C₆-Alkylgruppe, eine C₂-C₆-Alkenylgruppe, eine C₁-C₆-Alkoxygruppe, ein Halogenatom (Cl, Br, J oder F), eine Hydroxygruppe, eine Aminogruppe, eine Carboxylgruppe, eine C₁-C₆-Alkylaminogruppe, eine C₁-C₆-Dialkylaminogruppe, eine Nitrogruppe, eine Nitrilgruppe, eine Hydroxy-C₁-C₆-alkyloxygruppe oder eine Ayclaminogruppe,
- n, m: stehen unabhängig voneinander für eine ganze Zahl von 1 -6,
- X⁻: steht für ein einwertiges Anion, vorzugsweise für Halogenid, Hydrogensulfat, ½ Sulfat, p-Benzolsulfonat, p-Toluolsulfonat, Acetat, Tetrafluoroborat, Trifluormethansulfonat, Hexafluorophosphat, Methansulfat oder Methansulfonat

in Mitteln zum Färben und gegebenenfalls gleichzeitigen Aufhellen menschlicher Haare zum Erhalt von Farbnuancen mit sehr guten Echtheitseigenschaften.

Bezüglich weiterer bevorzugter erfindungsgemäßer Verwendungen gilt mutatis mutandis das zu den bevorzugten Mitteln Ausgeführte.

Einige der in den erfindungsgemäßen Mitteln enthaltenen kationischen Verbindungen sind bislang nicht bekannt.

Weiterhin sind die folgenden Verbindungen nicht literaturbekannt und Gegenstand der vorliegenden Erfindung
- Salze des 1-[2-({4-[2-(4,5-Dicyanoimidazol-2-yl)diazenyl]phenyl}ethylamino)ethyl]-4-aza-1-azoniabicyclo[2.2.2]octans
- Salze des 1-[2-({4-[2-(4,5-Dicyanoimidazol-2-yl)diazenyl]-3-methylphenyl}ethylamino)ethyl]-4-aza-1-azoniabicyclo[2.2.2]octans
- Salze des 1-[2-({4-[2-(4,5-Dicyanoimidazol-2-yl)diazenyl]-3-chlorphenyl}ethylamino)ethyl]-4-aza-1-azoniabicyclo[2.2.2]octans
- Salze des 1-[2-({4-[2-(1,2,4-4H-Triazol-3-yl)diazenyl]phenyl}ethylamino)ethyl]-4-aza-1-azoniabicyclo[2.2.2]octans
- Salze des 1-[2-({4-[2-(1,2,4-4H-Triazol-3-yl)diazenyl]-3-methylphenyl}ethylamino)ethyl]-4-aza-1-azoniabicyclo[2.2.2]octans
- Salze des 1-[2-({4-[2-(1,2,4-4H-Triazol-3-yl)diazenyl]-3-chlorphenyl}ethylamino)ethyl]-4-aza-1-azoniabicyclo-[2.2.2]octans
- Salze des 1-[2-({4-[2-(5-Phenyl-1H-pyrazol-3-yl)diazenyl]phenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-methylphenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-chlorphenyl}ethylamino)ethyl]-4-aza-1-azoniabicyclo-[2.2.2]octans
- Salze des 1-[2-({4-[2-(Thiazol-2-yl)diazenyl]phenyl}ethylamino)ethyl]-4-aza-1-azoniabicyclo[2.2.2]octans
- Salze des 1-[2-({4-[2-(Thiazol-2-yl)diazenyl]-3-methylphenyl}ethylamino)ethyl]-4-aza-1-azoniabicyclo[2.2.2]octans
- Salze des 1-[2-({4-[2-(Thiazol-2-yl)diazenyl]-3-chlorphenyl}ethylamino)ethyl]-4-aza-1-azoniabicyclo-[2.2.2]octans
- Salze des 1-[2-({4-[2-(5-Nitrothiazol-2-yl)diazenyl]phenyl}ethylamino)ethyl]-4-aza-1-azoniabicyclo[2.2.2]octans
- Salze des 1-[2-({4-[2-(5-Nitrothiazol-2-yl)diazenyl]-3-methylphenyl}ethylamino)ethyl]-4-aza-1-azoniabicyclo[2.2.2]octans
- Salze des 1-[2-({4-[2-(5-Nitrothiazol-2-yl)diazenyl]-3-chlorphenyl}ethylamino)ethyl]-4-aza-1-azoniabicyclo-[2.2.2]octans
- Salze des 1-[2-({4-[2-(3-Phenyl-1,2,4-thiadiazol-5-yl)diazenyl]phenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-methylphenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-chlorphenyl}ethylamino)ethyl]-4-aza-1-azoniabicyclo-[2.2.2]octans
- Salze des 1-[2-({4-[2-(5-Nitro-1H-benzimidazol-2-yl)diazenyl]phenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-methylphenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-chlorphenyl}ethylamino)ethyl]-4-aza-1-azoniabicyclo-[2.2.2]octans
- Salze des 1-[2-({4-[2-(5-Nitro-benzothiazol-2-yl)diazenyl]phenyl}ethylamino)ethyl]-4-aza-1-azoniabicyclo[2.2.2]octans
- Salze des 1-[2-({4-[2-(5-Nitro-benzothiazol-2-yl)diazenyl]-3-methylphenyl}ethylamino)ethyl]-4-aza-1-azoniabicyclo[2.2.2]octans
- Salze des 1-[2-({4-[2-(5-Nitro-benzothiazol-2-yl)diazenyl]-3-chlorphenyl}ethylamino)ethyl]-4-aza-1-azoniabicyclo-[2.2.2]octans
- Salze des 1-{2-[(4-{2-[6-(Methylsulfonyl)benzothiazol-2-yl]diazeny}phenyl)-ethylamino]ethyl}-4-aza-1-azoniabicyclo[2.2.2]octans
- Salze des 1-{2-[(4-{2-[6-(Methylsulfonyl)benzothiazol-2-yl]diazeny}-3-methylphenyl)-ethylamino]ethyl}-4-aza-1-azoniabicyclo[2.2.2]octans
- Salze des 1-{2-[(4-{2-[6-(Methylsulfonyl)benzothiazol-2-yl]diazenyl-3-chlorphenyl)-ethylamino]ethyl}-4-aza-1-azoniabicyclo[2.2.2]octans
- Salze des 1-[2-({4-[2-(5-Methoxybenzothiazol-2-yl)diazenyl]phenyl}ethylamino)ethyl]-4-aza-1-azoniabicyclo[2.2.2]octans
- Salze des 1-[2-({4-[2-(5-Methoxybenzothiazol-2-yl)diazenyl]-3-methylphenyl}ethylamino)ethyl]-4-aza-1-azoniabicyclo[2.2.2]octans
- Salze des 1-[2-({4-[2-(5-Methoxybenzothiazol-2-yl)diazenyl]-3-chlorphenyl}ethylamino)ethyl]-4-aza-1-azoniabicyclo-[2.2.2]octans
- Salze des 1-[2-({4-[2-(1H-Benzotriazol-6-yl)diazenyl]phenyl}ethylamino)ethyl]-4-aza-1-azoniabicyclo[2.2.2]octans
- Salze des 1-[2-({4-[2-(1H-Benzotriazol-6-yl)diazenyl]-3-methylphenyl}ethylamino)ethyl]-4-aza-1-azoniabicyclo[2.2.2]octans
- Salze des 1-[2-({4-[2-(1H-Benzotriazol-6-yl)diazenyl]-3-chlorphenyl}ethylamino)ethyl]-4-aza-1-azoniabicyclo[2.2.2]octans.

### Beispiele

### 1 Synthesebeispiele:

### Synthesebeispiel 1: Synthese von 1-[2-({4-[2-(2-Cyano-4-nitrophenyl)diazenyl]-phenyl}ethylamino)ethyl]-4-aza-1-azoniabicyclo[2.2.2]octan, Chlorid (DZ 1)

### 1.1. Synthese von N-(2-Chlorethyl)-N-ethylanilin

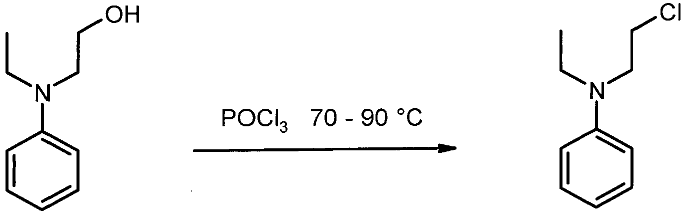

76,6 g (0,5 mol) Phosphoroxychlorid wurden vorgelegt und auf 80 °C erwärmt. Hierzu wurden 82,6 g (0,5 mol) N-(2-Hydroxyethyl)-N-ethylanilin getropft (exotherme Reaktion). Nach Beendigung des Zutropfens wurde für eine Stunde bei 80 °C nachgerührt. Nach dem Abkühlen wurde das Reaktionsgemisch auf 300 ml Eiswasser gegossen. Mit 530 ml einer 10 prozentigen, wässrigen Natronlaugelösung wurde ein pH-Wert von 5 eingestellt. Hierbei schied sich ein braunes Öl ab, welches abgetrennt und bei einem Vakuum von 30 mbar destilliert wurde (Übergangstemperatur 142 °C). Das Produkt fiel in Form eines farblosen Öls an. Ausbeute: 64,1 g (70,0 %)
¹H-NMR (400 MHz, DMSO-d₆): δ [ppm] = 1,11 (t, 3H); 3,38 (q, 2H); 3,64 (t, 2H); 3,70 (t, 2H); 6,61 (m, 1H); 6,69 (m, 2H); 7,15 (m, 2H)
¹³C-NMR (400 MHz, DMSO-d6): δ [ppm] = 12,1; 41,3; 44,2; 51,7; 111,6; 116,0; 129,3; 147,2

### 1.2. Synthese von 1-[2-(N-Methyl-N-phenylamino)ethyl]-4-aza-1-azoniabicyclo[2.2.2]octan, Chlorid

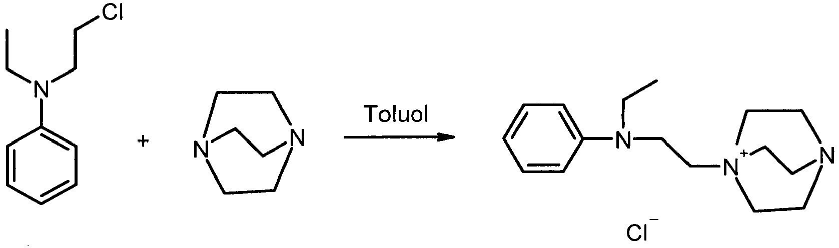

23,1 g (0,20 mol) 1,4-Diazabicyclo[2.2.2]octan wurden in 400 ml Toluol gelöst und bei Raumtemperatur mit 32,8 g (0,178 mol) N-(2-Chlorethyl)-N-ethylanilin aus Stufe 1.1 versetzt. Dieses Gemisch wurde für 72 Stunden bei einer Ölbadtemperatur von 90 °C gerührt. Nach jeweils ca. 24 Stunden ließ man das Reaktionsgemisch abkühlen und filtrierte den entstandenen Niederschlag ab. Die verschiedenen Fraktionen des Niederschlags wurden vereinigt, mit Methyl-*tert*-butylether nachgewaschen und bei 50 °C im Vakuum getrocknet. Ausbeute: 17,4 g (32,9 %)
¹H-NMR (400 MHz, DMSO-d₆): δ [ppm] = 1,12 (t, 3H); 3,33 (m, 6H); 3,41 (m, 4H); 3,48 (m, 6H); 3,80 (t, 2H);6,95 (m, 3H); 7,39 (m, 2H)
¹³C-NMR (400 MHz, DMSO-d6): δ [ppm] = 14,0; 45,8; 47,1; 48,6; 55,2; 63,0; 118,6; 121,9; 132,9; 150,0

### 1.3. Synthese von 1-[2-({4-[2-(2-Cyano-4-nitrophenyl)diazenyl]phenyl}ethylamino)ethyl]-4-aza-1-azoniabicyclo[2.2.2]octan, Chlorid (DZ 1)

### Diazotierung

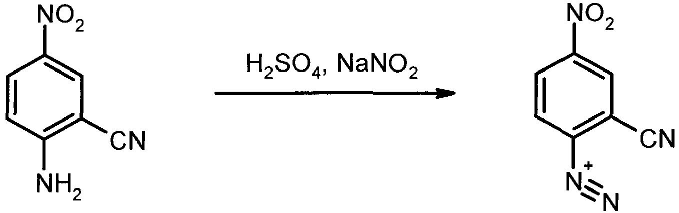

3,92 g (0,057 mol) Natriumnitrit wurden unter Kühlung innerhalb von 45 Minuten in 82 g (44,5 ml) konzentrierte Schwefelsäure gegeben. Zu dieser Mischung wurden unter Kühlung auf 0 bis 3 °C 9,0 g (0,055 mol) 2-Amino-5-nitrobenzonitril hinzugefügt. Unter Zuhilfenahme eines mit Eiswasser gefüllten Ultraschallbades wurde für 2 Stunden nachgerührt. Die auf diese Weise erhaltene orange Suspension wurde auf 220 g Eis gegossen. Durch Zugabe von 0,55 g (0,0056 mol) Amidosulfonsäure wurde das überschüssige Natriumnitrit zerstört. Diese Diazoniumsalzlösung wurde filtriert und dann unter Kühlung zur Kupplungskomponente gegeben.

### Azokupplung

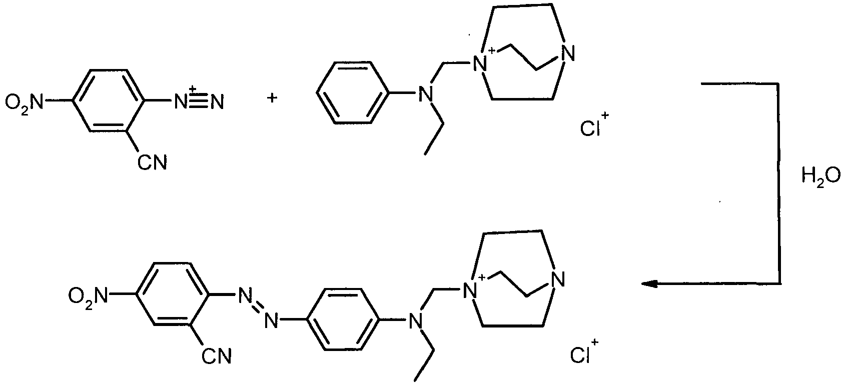

17,75 g (0,06 mol) 1-[2-(N-Methyl-N-phenylamino)ethyl]-4-aza-1-azoniabicyclo-[2.2.2]octan, Chlorid aus Stufe 1.2. wurden in 200 ml Wasser (dest.) gelöst und auf 0 bis 5 °C gekühlt. Unter Kühlung wurde die zuvor hergestellte Lösung des Diazoniumsalzes hinzugetropft. Die Reaktionslösung färbte sich dunkel violett. Anschießend wurde zunächst unter Kühlung, dann bei Raumtemperatur für einige Stunden nachgerührt. Durch Zugabe von 262 ml einer 10 prozentigen Natronlaugelösung wurde ein pH-Wert von 7 eingestellt. Dann wurde das Reaktionsgemisch am Rotattionsverdampfer zur Hälfte eingeengt und über Nacht stehen gelassen, woraufhin sich ein violettes Öl abschied. Das Öl wurde wiederholt mit je 600 ml Ethanol extrahiert. Nach der Extrakion mit Ethanol blieb ein violetter Feststoff zurück, bei welchem es sich um das gewünschte Produkt handelte. Ausbeute: 27,6 g (97 %) ¹H-NMR (400 MHz, DMSO-d₆): δ [ppm] = 1,22 (t, 3H); 3,09 (m, 6H); 3,51 (m, 8H); 3,63 (q, 2H); 3,98 (q, 2H); 7,03 (d, 2H); 7,95 (d, 2H); 7,98 (d, 1H); 8,58 (d, 1H); 8,87 (s, 1H)

### Synthesebeispiel 2: Synthese von 1-[2-({4-[2-(5-Nitrothiazol-2-yl)diazenyl]phenyl}ethylamino)-ethyl]-4-aza-1-azoniabicyclo[2.2.2]octan, Chlorid (DZ 2)

### 2.1. Synthese von N-(2-Chlorethyl)-N-ethylanilin

vgl. Synthesebeispiel 1.1.

### 2.2. Synthese von 1-[2-(N-Methyl-N-phenylamino)ethyl]-4-aza-1-azoniabicyclo[2.2.2]octan, Chlorid

vgl. Synthesebeispiel 1.2.

### 2.3. Synthese von1-[2-({4-[2-(5-Nitrothiazol-2-yl)diazenyl]phenyl}ethylamino)-ethyl]-4-aza-1-azoniabicyclo[2.2.2]octan, Chlorid (DZ 2)

### Diazotierung

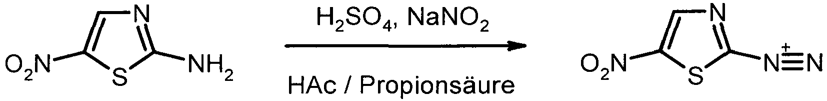

1,52 g (0,02 mol) Natriumnitrit wurden unter Kühlung innerhalb von 45 Minuten in 10 ml konzentrierte Schwefelsäure gegeben. Hierzu wurden bei 5 °C 20 ml eines Gemisches aus Propionsäure und Essigsäure (1:6) getropft. Dieses Gemisch wurde dann unter Kühlung auf 0 bis 3 °C zu einer Mischung aus 2,90 g (0,02 mol) 2-Amino-5-nitrothiazol in 20 ml Propionsäure/Essigsäure (1:6) getropft. Bei 5 °C wurde für eine Stunde nachgerührt. Anschließend wurde der Überschuß an Natriumnitrit durch Zugabe einer kleinen Menge Harnstoff zerstört.

### Azokupplung

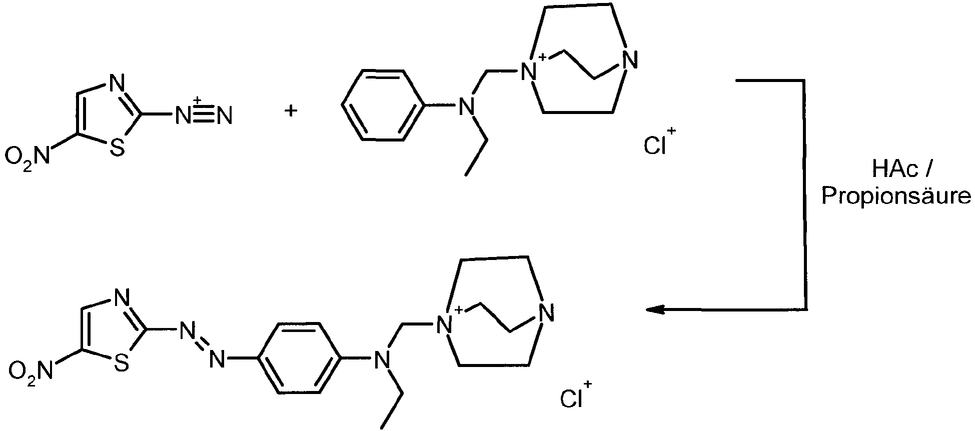

5,91 g (0,02 mol) 1-[2-(N-Methyl-N-phenylamino)ethyl]-4-aza-1-azoniabicyclo-[2.2.2]octan, Chlorid aus Stufe 2.2. wurden in 21 ml eines Gemisches aus Propionsäure und Essigsäure (1:6) gelöst. Diese Lösung wurde bei 0 bis 5 °C zur Suspension des Diazoniumsalzes getropft. Nach Beendigung des Zutropfens wurde für 1 Stunde bei 5 °C nachgerührt. Dann wurde das Reaktionsgemisch mit 200 ml Wasser versetzt und mit einer 50 prozentigen wässrigen Natriumhydroxid-Lösung auf einen pH-Wert von 7 gestellt. Der hierbei ausfallende violettblaue Feststoff wurde abfiltriert, zweimal mit wenig Wasser (ca. 150 ml) gewaschen und im Vakuum getrocknet.
Ausbeute: 2,7 g (30 %)
¹H-NMR (400 MHz, DMSO-d₆): δ [ppm] = 1,25 (t, 3H); 3,07 (m, 6H); 3,49 (m, 8H); 3,64 (q, 2H); 4,04 (q, 2H); 7,05 (d, 2H); 7,89 (d, 2H); 8,87 (s, 1H)

### 2 Ausfärbungsbeispiele:

### 2.1 Herstellung der Färbecremes

Es wurde folgende Färbecremes hergestellt:

### 2.1.1 Nichtionische Färbecreme

| | |
|---|---|
| Hydrenol® D | 6,0 g |
| Lorol® (techn.) | 6,0 g |
| Eumulgin® RH 40 | 1,0 g |
| Eumulgin® B1 | 3,0 g |
| Eumulgin® B2 | 3,0 g |
| PHB-Methylester | 0,3 g |
| PHB-Propylester | 0,2 g |
| Phenoxyethanol | 1,0 g |
| Polydiol® 400 | 5,0 g |
| Direktzieher | 1,0 g |
| Ammoniumsulfat | 1,0 g (in 30,0 g Wasser) |
| Natrosol® 250 HR | 1,0 g (in 15,0 g Wasser) |
| NaOH 0,1% | pH-Wert Einstellung |
| Wasser | ad 100 g |

Die ersten neun Komponenten wurden zusammen bei 80 °C aufgeschmolzen, danach wurde der Farbstoff zugefügt. Diese Mischung wurde mit einer Lösung des Ammoniumsulfats in 30 g Wasser emulgiert. Anschließend wurde eine Quellung von 1,0 g Natrosol® 250 HR in 15,0 g Wasser hinzugegeben. Der in der Tabelle 2 angegebene pH-Wert wurde mit 0,1% Natronlauge eingestellt, anschließend wurde mit Wasser auf 100 g aufgefüllt.

### 2.1.2 Kationische Färbecreme

| | |
|---|---|
| Stenol® 16/18 | 4,0 g |
| Eumulgin® B1 | 1,0 g |
| Dehyquart® A-CA | 2,0 g |
| Ammoniumsulfat | 1,0 g (in 30,0 g Wasser) |
| Direktzieher | 1,0 g |
| Wasser | at 100 g |

Das Stenol® 16/18 wurde zusammen mit Eumulgin® B1 und Dehyquart® A-CA aufgeschmolzen, danach wurde die Schmelze mit heißem Wasser emulgiert. Dann wurden der Farbstoff sowie die wässrige Ammoniumsulfatlösung hinzugegeben. Der pH-Wert wurde mit Ammoniak bwz. Zitronensäure auf den in der Tabelle angegebenen Wert eingestellt, anschließend wurde mit Wasser wurde auf 100 g aufgefüllt.

### 2.1.3 Anionische Färbecreme

| | |
|---|---|
| Hydrenol® D | 1,0 g |
| Lorol® techn. | 1,0 g |
| Akypo Soft® RLM 45N | 1,1 g |
| PHB-Propylester | 0,1 g |
| PHB-Methylester | 0,1 g |
| Ammoniumsulfat | 1,0 g (in 30,0 g Wasser) |
| Direktzieher DZ | 1,0 g |
| Wasser | at 100g |

Die ersten fünf Komponenten wurden zusammen aufgeschmolzen. Diese Schmelze wurde mit heißem Wasser emulgiert, anschließend wurde der in Wasser vorgelöste bzw. vordispergierte Farbstoff hinzugegeben sowie die Ammoniumsulfatlösung hinzugefügt. Der in der Tabelle angegebene pH-Wert wurde mit Ammoniak bzw. Zitronensäuer eingestellt, danach wurde mit Wasser auf 100 g aufgefüllt.

### 2.2 Verzeichnis der eingesetzten Rohstoffe

| | |
|---|---|
| Akypo RLM 45 NV® | Laurylalkohol-4.5-EO-Essigsäure-Natrium-Salz (mind. 22% Aktivsubstanzgehalt; INCI-Bezeichnung: Sodium Laureth-5 -Carboxylate) (Chem-Y) |
| Dehyquart® A-CA | Trimethylhexadecylammoniumchlorid (ca. 24 -26 % Aktivsubstanz; INCI-Bezeichnung: Aqua (Water), Cetrimonium Chloride) (Cognis) |
| Eumulgin® B1 | Cetylstearylalkohol mit ca. 12-EO-Einheiten (INCI-Bezeichnung: Ceteareth-12) (Cognis) |
| Eumulgin® B2 | Cetylstearylalkohol mit ca. 20 EO-Einheiten (INCI-Bezeichnung: Ceteareth-20) (Cognis) |
| Eumulgin® RH 40 | gehärtetes Rizinusöl mit ca. 40-EO-Einheiten (INCI-Bezeichnung: PEG-40 Hydrogenated Castor Oil) (Cognis) |
| Hydrenol® D | C₁₆₋₁₈-Fettalkohol (INCI-Bezeichnung: Cetearyl alcohol) (Cognis) |
| Lorol (techn.)® | C₁₂₋₁₈-Fettalkohol (INCI-Bezeichnung: Coconut Alcohol) (Cognis) |
| Natrosol® 250 HR | Hydroxyethylcellulose (INCI-Bezeichnung: Hydroxyethylcellulose) (Hercules) |
| Polydiol® 400 | Polyethylenglykol (INCI-Bezeichung: PEG-8) (Cognis) |
| Stenol® 1618 | C₁₆₋₁₈-Fettalkohol (INCI-Bezeichnung: Cetearyl Alcohol) (Cognis) |

### 2.3 Ausfärbungen

Jeweils 1,8 g der Färbecreme wurden auf eine ca. 6 cm lange Strähne Menschenhaares (Kerling Euronaturhaar, blond) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Die Haarsträhnen wurden in den in Tabelle 2 angegebenen Nuancen gefärbt.

### Tabelle 2:

### DZ1: 1-[2-({4-[2-(2-Cyano-4-nitrophenyl)diazenyl]-phenyl}ethylamino)ethyl]-4-aza-1-azoniabicyclo[2.2.2]octan, Chlorid

### DZ2: 1-[2-({4-[2-(5-Nitrothiazol-2-yl)diazenyl]phenyl}ethylamino)-ethyl]-4-aza-1-azoniabicyclo[2.2.2]octan, Chlorid (DZ 2)

| **Farbstoff** | **Färbecreme** | **pH-Wert** | **Farbnuance (Intensität)** |
|---|---|---|---|
| DZ 1 | 1 | 7,0 | graurubin (+++) |
| DZ 1 | 1 | 9,0 | portweinrot (+++) |
| DZ 1 | 2 | 7,0 | portweinrot (+++) |
| DZ 1 | 2 | 9,0 | portweinrot (+++) |
| DZ 1 | 3 | 7,0 | graurubin (+++) |
| DZ 1 | 3 | 9,0 | rubin (+++) |
| DZ 2 | 1 | 6,9 | mattviolett (+++) |
| DZ 2 | 1 | 9,1 | dunkelviolett (+++) |
| DZ 2 | 2 | 7,0 | dunkelviolett (+++) |
| DZ 2 | 2 | 9,1 | dunkelviolett (+++) |
| DZ 2 | 3 | 7,1 | violett (+++) |
| DZ 2 | 3 | 8,9 | tiefviolett (+++) |

| | | | |
|---|---|---|---|
| Intensität: + = niedrig ++ = mittel +++ = hoch | | | |

### 2.4 Überprüfung der Waschechtheit

Die unter 2.2. erhaltene Färbecreme 2 wurde auf Egalisiersträhnen ausgefärbt. Egalisiersträhnen sind Haarsträhnen (Kerling, 80 % grau) mit unterschiedlichem Schädigungsgrad, die im oberen Strähnenbereich mit einer Ultrablondierung und im unteren Strähnenbereich jeweils zweimal abwechselnd mit einer Dauerwelle und einer Ultrablondierung behandelt wurden. Auf diese Weise enthalten Egalisiersträhnen einen moderat geschädigten oberen und einen stark geschädigten unteren Strähnenteil.

Zur Bestimmung der Waschechheit wurden die Egalisiersträhnen 6 mal per Hand gewaschen. Hierzu wurde die Strähne zunächst mit Wasser angefeuchtet und anschließend jeweils für 45 Sekunden mit einer 25 %igen Texapon NSO-UP Lösung shampooniert. Das shampoonierte Haar wurde eine Minute lang mit handwarmem Wasser gründlich ausgespült. Anschließend wurde die Haarsträhne mit einem Fön gertrocknet. Nach dem Trocknen erfolgte der nächste Waschvorgang. Die Strähnen wurden nach 0, 1 und 6 Haarwäschen jeweils im oberen und unteren Strähnenteil farbmetrisch vermessen.

Aus der Tabelle ist ersichtlich, dass sich die Lab-Werte insbesondere im stark geschädigten, unteren Strähnenteil nach 6 Haarwäschen nur wenig verändern.

| **DZ1: 1-[2-({4-[2-(2-Cyano-4-nitrophenyl)diazenyl]-phenyl}ethylamino)ethyl]-4-aza-1-azoniabicyclo[2.2.2]octan, Chlorid** | oberer Strähnenteil | | | unterer Strähnenteil | | |
|---|---|---|---|---|---|---|
| | L | a | b | L | a | b |
| 0 Wäschen | 21,93 | 14,82 | 5,76 | 15,23 | 4,49 | 0,94 |
| 1 Haarwäsche | 24,94 | 25,36 | 3,73 | 17,72 | 4,41 | 3,30 |
| 6 Haarwäschen | 31,65 | 31,63 | 2,59 | 18,59 | 11,25 | 3,72 |

## Patentansprüche

1. Mittel zum Färben und gegebenenfalls gleichzeitigen Aufhellen von menschlichen Haaren, enthaltend mindestens eine kationische Verbindung der Formel (I): 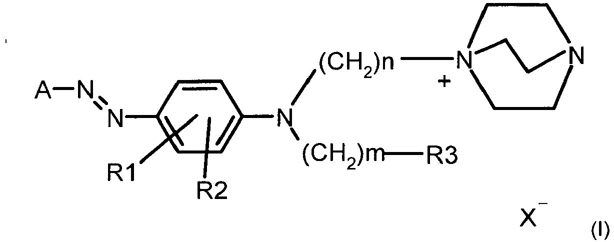 in der
A für einen gegebenenfalls substituierten Benzenring oder einen gegebenenfalls substituierten Naphthalenring der allgemeinen Formeln (II) oder (III) steht: 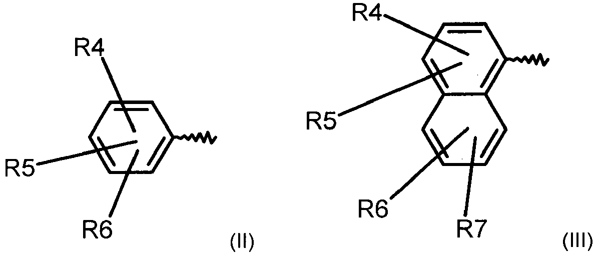 in denen
R⁴, R⁵, R⁶, R⁷ unabhängig voneinander für ein Wasserstoffatom, eine Nitrogruppe, eine Sulfonsäuregruppe, eine Nitrilgruppe, ein Halogenatom (Chlorid, Bromid oder Fluorid), eine Carbamoylgruppe, eine C₁-C₆-Alkylsulfonylgruppe, eine Sulfamoylgruppe oder eine C₁-C₆-Alkoxygruppe stehen, oder
A für einen der nachfolgend aufgeführten Heterocyclen (IV) bis (XV) steht 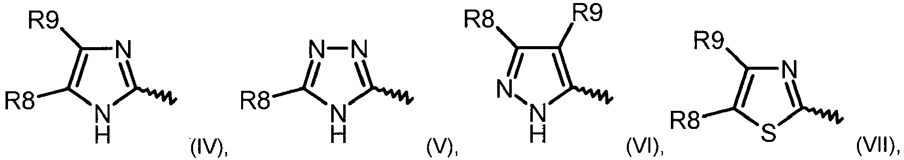 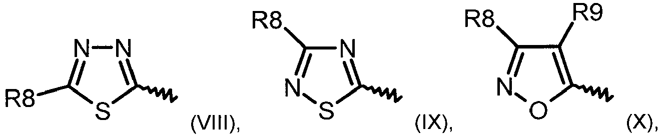 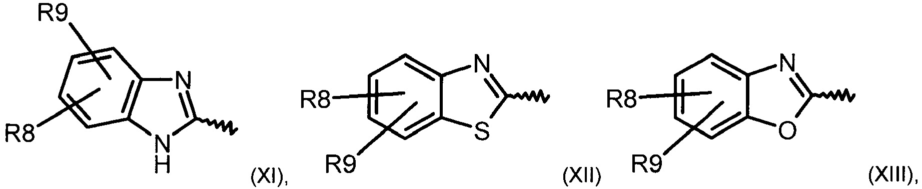 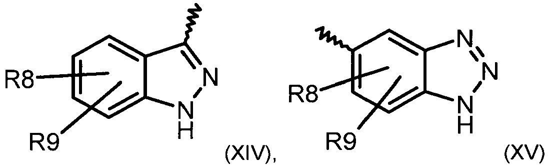
in denen
R⁸, R⁹ unabhängig voneinander für ein Wasserstoffatom, eine Nitrogruppe, eine Nitrilgruppe, ein Halogenatom, eine C₁-C₆-Alkylsulfonylgruppe, eine Carbamoylgruppe, eine C₁-C₆-Alkylgruppe, eine C₁-C₆-Alkoxygruppe oder eine Phenylgruppe stehen,
R¹, R², R³ stehen unabhängig voneinander für ein Wasserstoffatom, eine C₁-C₆-Alkylgruppe, eine C₂-C₆-Alkenylgruppe, eine C₁-C₆-Alkoxygruppe, ein Halogenatom (Cl, Br, J oder F), eine Hydroxygruppe, eine Aminogruppe, eine Carboxylgruppe, eine C₁-C₆-Alkylaminogruppe, eine C₁-C₆-Dialkylaminogruppe, eine Nitrogruppe, eine Nitrilgruppe, eine Hydroxy-C₁-C₆-alkyloxygruppe oder eine Ayclaminogruppe,
n, m stehen unabhängig voneinander für eine ganze Zahl von 1 - 6,
X⁻ steht für ein einwertiges Anion, vorzugsweise für Halogenid, Hydrogensulfat, ½ Sulfat, p-Benzolsulfonat, p-Toluolsulfonat, Acetat, Tetrafluoroborat, Trifluormethansulfonat, Hexafluorophosphat, Methansulfat oder Methansulfonat.

2. Mittel zum Färben und gegebenenfalls gleichzeitigen Aufhellen menschlicher Haare, nach Anspruch 1, **dadurch gekennzeichnet, dass** es mindestens eine kationische Verbindung aus der Gruppe der
- Salze des 1-[2-({4-[2-(4-Nitrophenyl)diazenyl]phenyl}ethylamino)ethyl]-4-aza-1-azoniabicyclo[2.2.2]octans
- Salze des 1-[2-({4-[2-(4-Nitrophenyl)diazenyl]-3-methylphenyl}ethylamino)ethyl]-4-aza-1-azoniabicyclo[2.2.2]octans
- Salze des 1-[2-({4-[2-(4-Nitrophenyl)diazenyl]-3-chlorphenyl}ethylamino)ethyl]-4-aza-1-azoniabicyclo[2.2.2]octans
- Salze des 1-[2-({4-[2-(2-Cyano-4-nitrophenyl)diazenyl]phenyl}ethylamino)ethyl]-4-aza-1-azoniabicyclo[2.2.2]octans
- Salze des 1-[2-({4-[2-(2-Cyano-4-nitrophenyl)diazenyl]-3-methylphenyl}ethylamino)ethyl]-4-aza-1-azoniabicyclo[2.2.2]octans
- Salze des 1-[2-({4-[2-(2-Cyano-4-nitrophenyl)diazenyl]-3-chlorphenyl}ethylamino)ethyl]-4-aza-1-azoniabicyclo[2.2.2]octans
- Salze des 1-[2-({4-[2-(2-Chlor-4-nitrophenyl)diazenyl]phenyl}ethylamino)ethyl]-4-aza-1-azoniabicyclo[2.2.2]octans
- Salze des 1-[2-({4-[2-(2-Chlor-4-nitrophenyl)diazenyl]-3-methylphenyl}ethylamino)ethyl]-4-aza-1-azoniabicyclo[2.2.2]octans
- Salze des 1-[2-({4-[2-(2-Chlor-4-nitrophenyl)diazenyl]-3-chlorphenyl}ethylamino)ethyl]-4-aza-1-azoniabicyclo[2.2.2]octans
- Salze des 1-[2-({4-[2-(2-Chlor-6-cyano-4-nitrophenyl)-diazenyl]phenyl}ethylamino)ethyl]-4-aza-1-azoniabicyclo[2.2.2]octans
- Salze des 1-[2-({4-[2-(2-Chlor-6-cyano-4-nitrophenyl)diazenyl]-3-methylphenyl}ethylamino)ethyl]-4-aza-1-azoniabicyclo[2.2.2]octans
- Salze des 1-[2-({4-[2-(2-Chlor-6-cyano-4-nitrophenyl)diazenyl]-3-chlorphenyl}ethylamino)ethyl]-4-aza-1-azoniabicyclo[2.2.2]octans
- Salze des 1-[2-({4-[2-(2,6-Dichlor-4-nitrophenyl)-diazenyl]phenyl}ethylamino)ethyl]-4-aza-1-azoniabicyclo[2.2.2]octans
- Salze des 1-[2-({4-[2-(2,6-Dichlor-4-nitrophenyl)diazenyl]-3-methylphenyl}ethylamino)ethyl]-4-aza-1-azoniabicyclo[2.2.2]octans
- Salze des 1-[2-({4-[2-(2,6-Dichlor-4-nitrophenyl)diazenyl]-3-chlorphenyl}ethylamino)ethyl]-4-aza-1-azoniabicyclo[2.2.2]octans
- Salze des 1-[2-({4-[2-(4-Methoxy-2-nitrophenyl)diazenyl]phenyl}ethylamino)ethyl]-4-aza-1-azoniabicyclo[2.2.2]octans
- Salze des 1-[2-({4-[2-(4-Methoxy-2-nitrophenyl)diazenyl]-3-methylphenyl}ethylamino)ethyl]-4-aza-1-azoniabicyclo[2.2.2]octans
- Salze des 1-[2-({4-[2-(4-Methoxy-2-nitrophenyl)diazenyl]-3-chlorphenyl}ethylamino)ethyl]-4-aza-1-azoniabicyclo[2.2.2]octans
- Salze des 1-[2-({4-[2-(2-Methoxy-4-nitrophenyl)diazenyl]phenyl}ethylamino)ethyl]-4-aza-1-azoniabicyclo[2.2.2]octans
- Salze des 1-[2-({4-[2-(2-Methoxy-4-nitrophenyl)diazenyl]-3-methylphenyl}ethylamino)ethyl]-4-aza-1-azoniabicyclo[2.2.2]octans
- Salze des 1-[2-({4-[2-(2-Methoxy-4-nitrophenyl)diazenyl]-3-chlorphenyl}ethylamino)ethyl]-4-aza-1-azoniabicyclo[2.2.2]octans
- Salze des 1-[2-({4-[2-(2-Methyl-4-nitrophenyl)diazenyl]phenyl}ethylamino)ethyl]-4-aza-1-azoniabicyclo[2.2.2]octans
- Salze des 1-[2-({4-[2-(2-Methyl-4-nitrophenyl)diazenyl]-3-methylphenyl}ethylamino)ethyl]-4-aza-1-azoniabicyclo[2.2.2]octans
- Salze des 1-[2-({4-[2-(2-Methyl-4-nitrophenyl)diazenyl]-3-chlorphenyl}ethylamino)ethyl]-4-aza-1-azoniabicyclo[2.2.2]octans
enthält.

3. Mittel zum Färben und gegebenenfalls gleichzeitigen Aufhellen menschlicher Haare nach einem der Ansprüche 1 bis 2, **dadurch gekennzeichnet, dass** es mindestens eine kationische Verbindung aus der Gruppe der
- Salze des 1-[2-({4-[2-(4,5-Dicyanoimidazol-2-yl)diazenyl]phenyl}ethylamino)ethyl]-4-aza-1-azoniabicyclo[2.2.2]octans
- Salze des 1-[2-({4-[2-(4,5-Dicyanoimidazol-2-yl)diazenyl]-3-methylphenyl}ethylamino)ethyl]-4-aza-1-azoniabicyclo[2.2.2]octans
- Salze des 1-[2-({4-[2-(4,5-Dicyanoimidazol-2-yl)diazenyl]-3-chlorphenyl}ethylamino)ethyl]-4-aza-1-azoniabicyclo[2.2.2]octans
- Salze des 1-[2-({4-[2-(1,2,4-4H-Triazol-3-yl)diazenyl]phenyl}ethylamino)ethyl]-4-aza-1-azoniabicyclo[2.2.2]octans
- Salze des 1-[2-({4-[2-(1,2,4-4H-Triazol-3-yl)diazenyl]-3-methylphenyl}ethylamino)ethyl]-4-aza-1-azoniabicyclo[2.2.2]octans
- Salze des 1-[2-({4-[2-(1,2,4-4H-Triazol-3-yl)diazenyl]-3-chlorphenyl}ethylamino)ethyl]-4-aza-1-azoniabicyclo-[2.2.2]octans
- Salze des 1-[2-({4-[2-(5-Phenyl-1H-pyrazol-3-yl)diazenyl]phenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-methylphenyl}ethylamino)ethyl]-4-aza-1-azoniabicyclo[2.2.2]octans
- Salze des 1-[2-({4-[2-(5-Phenyl-1H-pyrazol-3-yl)diazenyl]-3-chlorphenyl}ethylamino)ethyl]-4-aza-1-azoniabicyclo-[2.2.2]octans
- Salze des 1-[2-({4-[2-(Thiazol-2-yl)diazenyl]phenyl}ethylamino)ethyl]-4-aza-1-azoniabicyclo[2.2.2]octans
- Salze des 1-[2-({4-[2-(Thiazol-2-yl)diazenyl]-3-methylphenyl}ethylamino)ethyl]-4-aza-1-azoniabicyclo[2.2.2]octans
- Salze des 1-[2-({4-[2-(Thiazol-2-yl)diazenyl]-3-chlorphenyl}ethylamino)ethyl]-4-aza-1-azoniabicyclo-[2.2.2]octans
- Salze des 1-[2-({4-[2-(5-Nitrothiazol-2-yl)diazenyl]phenyl}ethylamino)ethyl]-4-aza-1-azoniabicyclo[2.2.2]octans
- Salze des 1-[2-({4-[2-(5-Nitrothiazol-2-yl)diazenyl]-3-methylphenyl}ethylamino)ethyl]-4-aza-1-azoniabicyclo[2.2.2]octans
- Salze des 1-[2-({4-[2-(5-Nitrothiazol-2-yl)diazenyl]-3-chlorphenyl}ethylamino)ethyl]-4-aza-1-azoniabicyclo-[2.2.2]octans
- Salze des 1-[2-({4-[2-(3-Phenyl-1,2,4-thiadiazol-5-yl)diazenyl]phenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-methylphenyl}ethylamino)ethyl]-4-aza-1-azoniabicyclo[2.2.2]octans
- Salze des 1-[2-({4-[2-(3-Phenyl-1,2,4-thiadiazol-5-yl)diazenyl]-3-chlorphenyl}ethylamino)ethyl]-4-aza-1-azoniabicyclo-[2.2.2]octans
- Salze des 1-[2-({4-[2-(5-Nitro-1H-benzimidazol-2-yl)diazenyl]phenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-methylphenyl}ethylamino)ethyl]-4-aza-1-azoniabicyclo[2.2.2]octans
- Salze des 1-[2-({4-[2-(5-Nitro-1H-benzimidazol-2-yl)diazenyl]-3-chlorphenyl}ethylamino)ethyl]-4-aza-1-azoniabicyclo-[2.2.2]octans
- Salze des 1-[2-({4-[2-(5-Nitro-benzothiazol-2-yl)diazenyl]phenyl}ethylamino)ethyl]-4-aza-1-azoniabicyclo[2.2.2]octans
- Salze des 1-[2-({4-[2-(5-Nitro-benzothiazol-2-yl)diazenyl]-3-methylphenyl}ethylamino)ethyl]-4-aza-1-azoniabicyclo[2.2.2]octans
- Salze des 1-[2-({4-[2-(5-Nitro-benzothiazol-2-yl)diazenyl]-3-chlorphenyl}ethylamino)ethyl]-4-aza-1-azoniabicyclo-[2.2.2]octans
- Salze des 1-{2-[(4-{2-[6-(Methylsulfonyl)benzothiazol-2-yl]diazeny}phenyl)-ethylamino]ethyl}-4-aza-1-azoniabicyclo[2.2.2]octans
- Salze des 1-{2-[(4-{2-[6-(Methylsulfonyl)benzothiazol-2-yl]diazeny}-3-methylphenyl)-ethylamino]ethyl}-4-aza-1-azoniabicyclo[2.2.2]octans
- Salze des 1-{2-[(4-{2-[6-(Methylsulfonyl)benzothiazol-2-yl]diazeny}-3-chlorphenyl)-ethylamino]ethyl}-4-aza-1-azoniabicyclo[2.2.2]octans
- Salze des 1-[2-({4-[2-(5-Methoxybenzothiazol-2-yl)diazenyl]phenyl}ethylamino)ethyl]-4-aza-1-azoniabicyclo[2.2.2]octans
- Salze des 1-[2-({4-[2-(5-Methoxybenzothiazol-2-yl)diazenyl]-3-methylphenyl}ethylamino)ethyl]-4-aza-1-azoniabicyclo[2.2.2]octans
- Salze des 1-[2-({4-[2-(5-Methoxybenzothiazol-2-yl)diazenyl]-3-chlorphenyl}ethylamino)ethyl]-4-aza-1-azoniabicyclo-[2.2.2]octans
- Salze des 1-[2-({4-[2-(1H-Benzotriazol-6-yl)diazenyl]phenyl}ethylamino)ethyl]-4-aza-1-azoniabicyclo[2.2.2]octans
- Salze des 1-[2-({4-[2-(1H-Benzotriazol-6-yl)diazenyl]-3-methylphenyl}ethylamino)ethyl]-4-aza-1-azoniabicyclo[2.2.2]octans
- Salze des 1-[2-({4-[2-(1H-Benzotriazol-6-yl)diazenyl]-3-chlorphenyl}ethylamino)ethyl]-4-aza-1-azoniabicyclo[2.2.2]octans
enthält.

4. Mittel zum Färben und gegebenenfalls gleichzeitigen Aufhellen menschlicher Haare nach einem der Ansprüche 1 bis 3, **dadurch gekennzeichnet, dass** es die Verbindung(en) der Formel (I) in Mengen von 0,001 bis 5 Gew.-%, vorzugsweise von 0,0025 bis 2,5 Gew.-%, besonders bevorzugt von 0,005 bis 1,5 Gew.-% und insbesondere von 0,01 bis 1 Gew.-%, jeweils bezogen auf das gesamte Mittel, enthält.

5. Mittel zum Färben und gegebenenfalls gleichzeitigen Aufhellen menschlicher Haare nach einem der Ansprüche 1 bis 4, **dadurch gekennzeichnet, dass** es zusätzlich - bezogen auf sein Gewicht - 0,001 bis 5 Gew.-% eines oder mehrerer Oxidationsfarbstoffvorprodukte und/oder direktziehender Farbstoffe enthält.

6. Mittel zum Färben und gegebenenfalls gleichzeitigen Aufhellen menschlicher Haare nach einem der Ansprüche 1 bis 5, **dadurch gekennzeichnet, dass** es 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthält.

7. Verfahren zum Färben und gegebenenfalls gleichzeitigen Aufhellen von menschlichen Haaren, **dadurch gekennzeichnet, dass**
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein Mittel nach einem der Ansprüche 1 bis 4 ist.

8. Verwendung von kationischen Verbindungen der Formel (I): 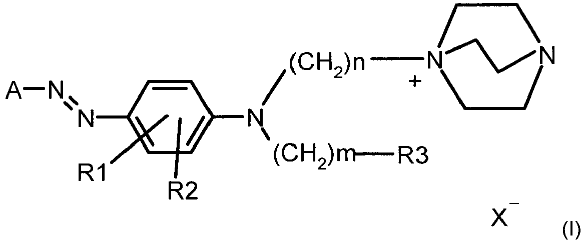 in der
A ein heterocyclisches oder carbocyclisches, gegebenenfalls substituiertes, ungeladenes Ringsystem ist,
R¹, R², R³ stehen unabhängig voneinander für ein Wasserstoffatom, eine C₁-C₆-Alkylgruppe, eine C₂-C₆-Alkenylgruppe, eine C₁-C₆-Alkoxygruppe, ein Halogenatom (Cl, Br, J oder F), eine Hydroxygruppe, eine Aminogruppe, eine Carboxylgruppe, eine C₁-C₆-Alkylaminogruppe, eine C₁-C₆-Dialkylaminogruppe, eine Nitrogruppe, eine Nitrilgruppe, eine Hydroxy-C₁-C₆-alkyloxygruppe oder eine Ayclaminogruppe,
n, m stehen unabhängig voneinander für eine ganze Zahl von 1 - 6,
X⁻ steht für ein einwertiges Anion, vorzugsweise für Halogenid, Hydrogensulfat, ½ Sulfat, p-Benzolsulfonat, p-Toluolsulfonat, Acetat, Tetrafluoroborat, Trifluormethansulfonat, Hexafluorophosphat, Methansulfat oder Methansulfonat
in Mitteln zum Färben und gegebenenfalls gleichzeitigen Aufhellen menschlicher Haare zum Erhalt von Farbnuancen mit sehr guten Echtheitseigenschaften.

9. Kationische Verbindungen der Formel (I) nach Anspruch 1 ausgewählt aus:
- Salzen des 1-[2-({4-[2-(4,5-Dicyanoimidazol-2-yl)diazenyl]phenyl}ethylamino)ethyl]-4-aza-1-azoniabicyclo[2.2.2]octans
- Salzen des 1-[2-({4-[2-(4,5-Dicyanoimidazol-2-yl)diazenyl]-3-methylphenyl}ethylamino)ethyl]-4-aza-1-azoniabicyclo[2.2.2]octans
- Salzen des 1-[2-({4-[2-(4,5-Dicyanoimidazol-2-yl)diazenyl]-3-chlorphenyl}ethylamino)ethyl]-4-aza-1-azoniabicyclo[2.2.2]octans
- Salzen des 1-[2-({4-[2-(1,2,4-4H-Triazol-3-yl)diazenyl]phenyl}ethylamino)ethyl]-4-aza-1-azoniabicyclo[2.2.2]octans
- Salzen des 1-[2-({4-[2-(1,2,4-4H-Triazol-3-yl)diazenyl]-3-methylphenyl}ethylamino)ethyl]-4-aza-1-azoniabicyclo[2.2.2]octans
- Salzen des 1-[2-({4-[2-(1,2,4-4H-Triazol-3-yl)diazenyl]-3-chlorphenyl}ethylamino)ethyl]-4-aza-1-azoniabicyclo-[2.2.2]octans
- Salzen des 1-[2-({4-[2-(5-Phenyl-1H-pyrazol-3-yl)diazenyl]phenyl}ethylamino)ethyl]-4-aza-1-azoniabicyclo[2.2.2]octans
- Salzen des 1-[2-({4-[2-(5-Phenyl-1H-pyrazol-3-yl)diazenyl]-3-methylphenyl}ethylamino)ethyl]-4-aza-1-azoniabicyclo[2.2.2]octans
- Salzen des 1-[2-({4-[2-(5-Phenyl-1H-pyrazol-3-yl)diazenyl]-3-chlorphenyl}ethylamino)ethyl]-4-aza-1-azoniabicyclo-[2.2.2]octans
- Salzen des 1-[2-({4-[2-(Thiazol-2-yl)diazenyl]phenyl}ethylamino)ethyl]-4-aza-1-azoniabicyclo[2.2.2]octans
- Salzen des 1-[2-({4-[2-(Thiazol-2-yl)diazenyl]-3-methylphenyl}ethylamino)ethyl]-4-aza-1-azoniabicyclo[2.2.2]octans
- Salzen des 1-[2-({4-[2-(Thiazol-2-yl)diazenyl]-3-chlorphenyl}ethylamino)ethyl]-4-aza-1-azoniabicyclo-[2.2.2]octans
- Salzen des 1-[2-({4-[2-(5-Nitrothiazol-2-yl)diazenyl]phenyl}ethylamino)ethyl]-4-aza-1-azoniabicyclo[2.2.2]octans
- Salzen des 1-[2-({4-[2-(5-Nitrothiazol-2-yl)diazenyl]-3-methylphenyl}ethylamino)ethyl]-4-aza-1-azoniabicyclo[2.2.2]octans
- Salzen des 1-[2-({4-[2-(5-Nitrothiazol-2-yl)diazenyl]-3-chlorphenyl}ethylamino)ethyl]-4-aza-1-azoniabicyclo-[2.2.2]octans
- Salzen des 1-[2-({4-[2-(3-Phenyl-1,2,4-thiadiazol-5-yl)diazenyl]phenyl}ethylamino)ethyl]-4-aza-1-azoniabicyclo[2.2.2]octans
- Salzen des 1-[2-({4-[2-(3-Phenyl-1,2,4-thiadiazol-5-yl)diazenyl]-3-methylphenyl}ethylamino)ethyl]-4-aza-1-azoniabicyclo[2.2.2]octans
- Salzen des 1-[2-({4-[2-(3-Phenyl-1,2,4-thiadiazol-5-yl)diazenyl]-3-chlorphenyl}ethylamino)ethyl]-4-aza-1-azoniabicyclo-[2.2.2]octans
- Salzen des 1-[2-({4-[2-(5-Nitro-1H-benzimidazol-2-yl)diazenyl]phenyl}ethylamino)ethyl]-4-aza-1-azoniabicyclo[2.2.2]octans
- Salzen des 1-[2-({4-[2-(5-Nitro-1H-benzimidazol-2-yl)diazenyl]-3-methylphenyl}ethylamino)ethyl]-4-aza-1-azoniabicyclo[2.2.2]octans
- Salzen des 1-[2-({4-[2-(5-Nitro-1H-benzimidazol-2-yl)diazenyl]-3-chlorphenyl}ethylamino)ethyl]-4-aza-1-azoniabicyclo-[2.2.2]octans
- Salzen des 1-[2-({4-[2-(5-Nitro-benzothiazol-2-yl)diazenyl]phenyl}ethylamino)ethyl]-4-aza-1-azoniabicyclo[2.2.2]octans
- Salzen des 1-[2-({4-[2-(5-Nitro-benzothiazol-2-yl)diazenyl]-3-methylphenyl}ethylamino)ethyl]-4-aza-1-azoniabicyclo[2.2.2]octans
- Salzen des 1-[2-({4-[2-(5-Nitro-benzothiazol-2-yl)diazenyl]-3-chlorphenyl}ethylamino)ethyl]-4-aza-1-azoniabicyclo-[2.2.2]octans
- Salzen des 1-{2-[(4-{2-[6-(Methylsulfonyl)benzothiazol-2-yl]diazeny}phenyl)-ethylamino]ethyl}-4-aza-1-azoniabicyclo[2.2.2]octans
- Salzen des 1-{2-[(4-{2-[6-(Methylsulfonyl)benzothiazol-2-yl]diazeny}-3-methylphenyl)-ethylamino]ethyl}-4-aza-1-azoniabicyclo[2.2.2]octans
- Salzen des 1-{2-[(4-{2-[6-(Methylsulfonyl)benzothiazol-2-yl]diazeny}-3-chlorphenyl)-ethylamino]ethyl}-4-aza-1-azoniabicyclo[2.2.2]octans
- Salzen des 1-[2-({4-[2-(5-Methoxybenzothiazol-2-yl)diazenyl]phenyl}ethylamino)ethyl]-4-aza-1-azoniabicyclo[2.2.2]octans
- Salzen des 1-[2-({4-[2-(5-Methoxybenzothiazol-2-yl)diazenyl]-3-methylphenyl}ethylamino)ethyl]-4-aza-1-azoniabicyclo[2.2.2]octans
- Salzen des 1-[2-({4-[2-(5-Methoxybenzothiazol-2-yl)diazenyl]-3-chlorphenyl}ethylamino)ethyl]-4-aza-1-azoniabicyclo-[2.2.2]octans
- Salzen des 1-[2-({4-[2-(1H-Benzotriazol-6-yl)diazenyl]phenyl}ethylamino)ethyl]-4-aza-1-azoniabicyclo[2.2.2]octans
- Salzen des 1-[2-({4-[2-(1H-Benzotriazol-6-yl)diazenyl]-3-methylphenyl}ethylamino)ethyl]-4-aza-1-azoniabicyclo[2.2.2]octans
- Salzen des 1-[2-({4-[2-(1H-Benzotriazol-6-yl)diazenyl]-3-chlorphenyl}ethylamino)ethyl]-4-aza-1-azoniabicyclo[2.2.2]octans

## Claims

1. An agent for dyeing and optionally simultaneously lightening human hair, containing at least one cationic compound of formula (I): 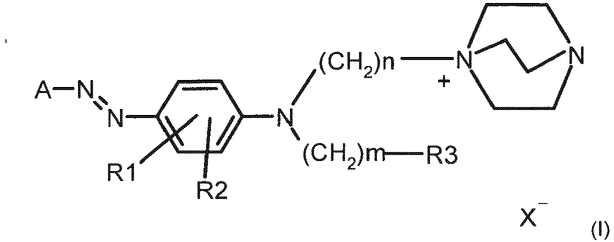 in which
A represents an optionally substituted benzene ring or an optionally substituted naphthalene ring of general formulas (II) or (III): 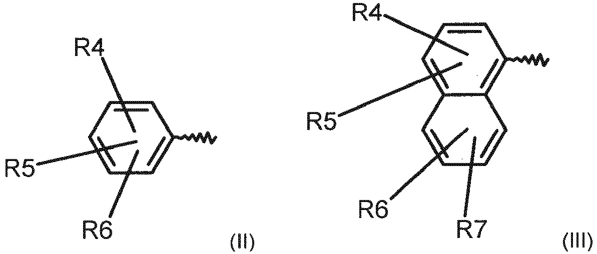
in which
R⁴, R⁵, R⁶, R⁷ represent, independently of one another, a hydrogen atom, a nitro group, a sulfonic acid group, a nitrile group, a halogen atom (chloride, bromide or fluoride), a carbamoyl group, a C₁-C₆ alkyl sulfonyl group, a sulfamoyl group or a C₁-C₆ alkoxy group, or
A represents one of the heterocycles (IV) to (XV) shown below 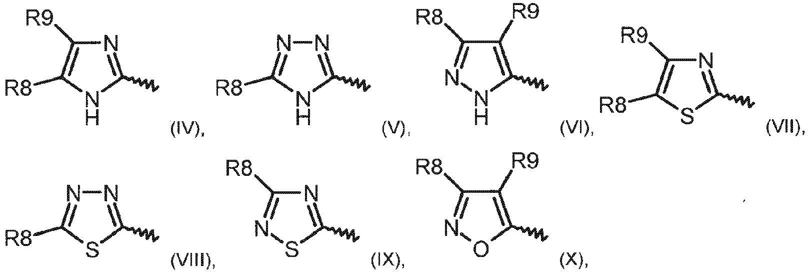 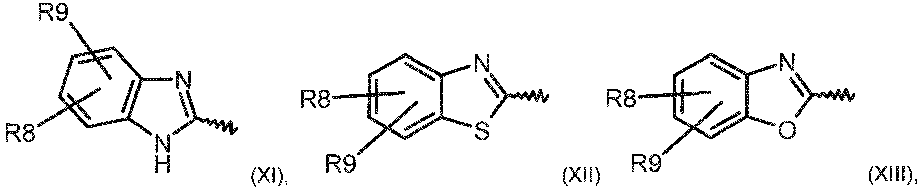 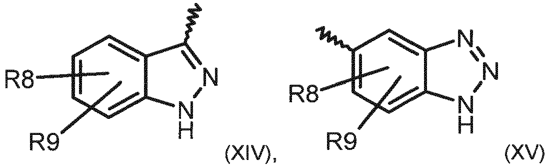
in which
R⁸, R⁹ represent, independently of one another, a hydrogen atom, a nitro group, a nitrile group, a halogen atom, a C₁-C₆ alkyl sulfonyl group, a carbamoyl group, a C₁-C₆ alkyl group, a C₁-C₆ alkoxy group or a phenyl group,
R¹, R², R³ represent, independently of one another, a hydrogen atom, a C₁-C₆ alkyl group, a C₂-C₆ alkenyl group, a C₁-C₆ alkoxy group, a halogen atom (CI, Br, J or F), a hydroxy group, an amino group, a carboxyl group, a C₁-C₆ alkylamino group, a C₁-C₆ dialkylamino group, a nitro group, a nitrile group, a hydroxy-C₁-C₆ alkyloxy group or an acylamino group,
n, m represent, independently of one another, an integer from 1 to 6,
X⁻ represents a monovalent anion, preferably halide, hydrogen sulfate, ½ sulfate, p-benzene sulfonate, p-toluene sulfonate, acetate, tetrafluoroborate, trifluoromethane sulfonate, hexafluorophosphate, methane sulfate or methane sulfonate.

2. The agent for dyeing and optionally simultaneously lightening human hair according to claim 1, **characterized in that** it contains at least one cationic compound from the group of
- salts of 1-[2-({4-[2-(4-nitrophenyl)diazenyl]phenyl}ethylamino)ethyl]-4-aza-1-azoniabicyclo[2.2.2]octane
- salts of 1-[2-({4-[2-(4-nitrophenyl)diazenyl]-3-methylphenyl}ethylamino)ethyl]-4-aza-1-azoniabicyclo[2.2.2]octane
- salts of 1-[2-({4-[2-(4-nitrophenyl)diazenyl]-3-chlorophenyl}ethylamino)ethyl]-4-aza-1-azoniabicyclo[2.2.2]octane
- salts of 1-[2-({4-[2-(2-cyano-4-nitrophenyl)diazenyl]phenyl}ethylamino)ethyl]-4-aza-1-azoniabicyclo[2.2.2]octane
- salts of 1-[2-({4-[2-(2-cyano-4-nitrophenyl)diazenyl]-3-methylphenyl}ethylamino)ethyl]-4-aza-1-azoniabicyclo[2.2.2]octane
- salts of 1-[2-({4-[2-(2-cyano-4-nitrophenyl)diazenyl]-3-chlorophenyl}ethylamino)ethyl]-4-aza-1-azoniabicyclo[2.2.2]octane
- salts of 1-[2-({4-[2-(2-chloro-4-nitrophenyl)diazenyl]phenyl}ethylamino)ethyl]-4-aza-1-azoniabicyclo[2.2.2]octane
- salts of 1-[2-({4-[2-(2-chloro-4-nitrophenyl)diazenyl]-3-methylphenyl}ethylamino)ethyl]-4-aza-1-azoniabicyclo[2.2.2]octane
- salts of 1-[2-({4-[2-(2-chloro-4-nitrophenyl)diazenyl]-3-chlorophenyl}ethylamino)ethyl]-4-aza-1-azoniabicyclo[2.2.2]octane
- salts of 1-[2-({4-[2-(2-chloro-6-cyano-4-nitrophenyl)-diazenyl]phenyl}ethylamino)ethyl]-4-aza-1-azoniabicyclo[2.2.2]octane
- salts of 1-[2-({4-[2-(2-chloro-6-cyano-4-nitrophenyl)diazenyl]-3-methylphenyl}ethylamino)ethyl]-4-aza-1-azoniabicyclo[2.2.2]octane
- salts of 1-[2-({4-[2-(2-chloro-6-cyano-4-nitrophenyl)diazenyl]-3-chlorophenyl}ethylamino)ethyl]-4-aza-1-azoniabicyclo[2.2.2]octane
- salts of 1-[2-({4-[2-(2,6-dichloro-4-nitrophenyl)-diazenyl]phenyl}ethylamino)ethyl]-4-aza-1-azoniabicyclo[2.2.2]octane
- salts of 1-[2-({4-[2-(2,6-dichloro-4-nitrophenyl)diazenyl]-3-methylphenyl}ethylamino)ethyl]-4-aza-1-azoniabicyclo[2.2.2]octane
- salts of 1-[2-({4-[2-(2,6-dichloro-4-nitrophenyl)diazenyl]-3-chlorophenyl}ethylamino)ethyl]-4-aza-1-azoniabicyclo[2.2.2]octane
- salts of 1-[2-({4-[2-(4-methoxy-2-nitrophenyl)diazenyl]phenyl}ethylamino)ethyl]-4-aza-1-azoniabicyclo[2.2.2]octane
- salts of 1-[2-({4-[2-(4-methoxy-2-nitrophenyl)diazenyl]-3-methylphenyl}ethylamino)ethyl]-4-aza-1-azoniabicyclo[2.2.2]octane
- salts of 1-[2-({4-[2-(4-methoxy-2-nitrophenyl)diazenyl]-3-chlorophenyl}ethylamino)ethyl]-4-aza-1-azoniabicyclo[2.2.2]octane
- salts of 1-[2-({4-[2-(2-methoxy-4-nitrophenyl)diazenyl]phenyl}ethylamino)ethyl]-4-aza-1-azoniabicyclo[2.2.2]octane
- salts of 1-[2-({4-[2-(2-methoxy-4-nitrophenyl)diazenyl]-3-methylphenyl}ethylamino)ethyl]-4-aza-1-azoniabicyclo[2.2.2]octane
- salts of 1-[2-({4-[2-(2-methoxy-4-nitrophenyl)diazenyl]-3-chlorophenyl}ethylamino)ethyl]-4-aza-1-azoniabicyclo[2.2.2]octane
- salts of 1-[2-({4-[2-(2-methyl-4-nitrophenyl)diazenyl]phenyl}ethylamino)ethyl]-4-aza-1-azoniabicyclo[2.2.2]octane
- salts of 1-[2-({4-[2-(2-methyl-4-nitrophenyl)diazenyl]-3-methylphenyl}ethylamino)ethyl]-4-aza-1-azoniabicyclo[2.2.2]octane
- salts of 1-[2-({4-[2-(2-methyl-4-nitrophenyl)diazenyl]-3-chlorophenyl}ethylamino)ethyl]-4-aza-1-azoniabicyclo[2.2.2]octane.

3. The agent for dyeing and optionally simultaneously lightening human hair according to one of claims 1 to 2, **characterized in that** it contains at least one cationic compound from the group of
- salts of 1-[2-({4-[2-(4,5-dicyanoimidazol-2-yl)diazenyl]phenyl}ethylamino)ethyl]-4-aza-1-azoniabicyclo[2.2.2]octane
- salts of 1-[2-({4-[2-(4,5-dicyanoimidazol-2-yl)diazenyl]-3-methylphenyl}ethylamino)ethyl]-4-aza-1-azoniabicyclo[2.2.2]octane
- salts of 1-[2-({4-[2-(4,5-dicyanoimidazol-2-yl)diazenyl]-3-chlorophenyl}ethylamino)ethyl]-4-aza-1-azoniabicyclo[2.2.2]octane
- salts of 1-[2-({4-[2-(1,2,4-4H-triazol-3-yl)diazenyl]phenyl}ethylamino)ethyl]-4-aza-1-azoniabicyclo[2.2.2]octane
- salts of 1-[2-({4-[2-(1,2,4-4H-triazol-3-yl)diazenyl]-3-methylphenyl}ethylamino)ethyl]-4-aza-1-azoniabicyclo[2.2.2]octane
- salts of 1-[2-({4-[2-(1,2,4-4H-triazol-3-yl)diazenyl]-3-chlorophenyl}ethylamino)ethyl]-4-aza-1-azoniabicyclo-[2.2.2]octane
- salts of 1-[2-({4-[2-(5-phenyl-1H-pyrazol-3-yl)diazenyl]phenyl}ethylamino)ethyl]-4-aza-1-azoniabicyclo[2.2.2]octane
- salts of 1-[2-({4-[2-(5-phenyl-1H-pyrazol-3-yl)diazenyl]-3-methylphenyl}ethylamino)ethyl]-4-aza-1-azoniabicyclo[2.2.2]octane
- salts of 1-[2-({4-[2-(5-phenyl-1H-pyrazol-3-yl)diazenyl]-3-chlorophenyl}ethylamino)ethyl]-4-aza-1-azoniabicyclo-[2.2.2]octane
- salts of 1[2-({4-[2-(thiazol-2-yl)diazenyl]phenyl}ethylamino)ethyl]-4-aza-1-azoniabicyclo[2.2.2]octane
- salts of 1-[2-({4-[2-(thiazol-2-yl)diazenyl]-3-methylphenyl}ethylamino)ethyl]-4-aza-1-azoniabicyclo[2.2.2]octane
- salts of 1-[2-({4-[2-(thiazol-2-yl)diazenyl]-3-chlorophenyl}ethylamino)ethyl]-4-aza-1-azoniabicyclo-[2.2.2]octane
- salts of 1-[2-({4-[2-(5-nitrothiazol-2-yl)diazenyl]phenyl}ethylamino)ethyl]-4-aza-1-azoniabicyclo[2.2.2]octane
- salts of 1-[2-({4-[2-(5-nitrothiazol-2-yl)diazenyl]-3-methylphenyl}ethylamino)ethyl]-4-aza-1-azoniabicyclo[2.2.2]octane
- salts of 1-[2-({4-[2-(5-nitrothiazol-2-yl)diazenyl]-3-chlorophenyl}ethylamino)ethyl]-4-aza-1-azoniabicyclo-[2.2.2]octane
- salts of 1-[2-({4-[2-(3-phenyl-1,2,4-thiadiazol-5-yl)diazenyl]phenyl}ethylamino)ethyl]-4-aza-1-azoniabicyclo[2.2.2]octane
- salts of 1-[2-({4-[2-(3-phenyl-1,2,4-thiadiazol-5-yl)diazenyl]-3-methylphenyl}ethylamino)ethyl]-4-aza-1-azoniabicyclo[2.2.2]octane
- salts of 1-[2-({4-[2-(3-phenyl-1,2,4-thiadiazol-5-yl)diazenyl]-3-chlorophenyl}ethylamino)ethyl]-4-aza-1-azoniabicyclo-[2.2.2]octane
- salts of 1-[2-({4-[2-(5-nitro-1H-benzimidazol-2-yl)diazenyl]phenyl}ethylamino)ethyl]-4-aza-1-azoniabicyclo[2.2.2]octane
- salts of 1-[2-({4-[2-(5-nitro-1H-benzimidazol-2-yl)diazenyl]-3-methylphenyl}ethylamino)ethyl]-4-aza-1-azoniabicyclo[2.2.2]octane
- salts of 1-[2-({4-[2-(5-nitro-1H-benzimidazol-2-yl)diazenyl]-3-chlorophenyl}ethylamino)ethyl]-4-aza-1-azoniabicyclo-[2.2.2]octane
- salts of 1-[2-({4-[2-(5-nitro-benzothiazol-2-yl)diazenyl]phenyl}ethylamino)ethyl]-4-aza-1-azoniabicyclo[2.2.2]octane
- salts of 1-[2-({4-[2-(5-nitro-benzothiazol-2-yl)diazenyl]-3-methylphenyl}ethylamino)ethyl]-4-aza-1-azoniabicyclo[2.2.2]octane
- salts of 1-[2-({4-[2-(5-nitro-benzothiazol-2-yl)diazenyl]-3-chlorophenyl}ethylamino)ethyl]-4-aza-1-azoniabicyclo-[2.2.2]octane
- salts of 1-{2-[(4-{2-[6-(methylsulfonyl)benzothiazol-2-yl]diazeny}phenyl)-ethylamino]ethyl}-4-aza-1-azoniabicyclo[2.2.2]octane
- salts of 1-{2-[(4-{2-[6-(methylsulfonyl)benzothiazol-2-yl]diazeny}-3-methylphenyl)-ethylamino]ethyl}-4-aza-1-azoniabicyclo[2.2.2]octane
- salts of 1-{2-[(4-{2-[6-(methylsulfonyl)benzothiazol-2-yl]diazeny}-3-chlorophenyl)-ethylamino]ethyl}-4-aza-1-azoniabicyclo[2.2.2]octane
- salts of 1-[2-({4-[2-(5-methoxybenzothiazol-2-yl)diazenyl]phenyl}ethylamino)ethyl]-4-aza-1-azoniabicyclo[2.2.2]octane
- salts of 1-[2-({4-[2-(5-methoxybenzothiazol-2-yl)diazenyl]-3-methylphenyl}ethylamino)ethyl]-4-aza-1-azoniabicyclo[2.2.2]octane
- salts of 1-[2-({4-[2-(5-methoxybenzothiazol-2-yl)diazenyl]-3-chlorophenyl}ethylamino)ethyl]-4-aza-1-azoniabicyclo-[2.2.2]octane
- salts of 1-[2-({4-[2-(1H-benzotriazol-6-yl)diazenyl]phenyl}ethylamino)ethyl]-4-aza-1-azoniabicyclo[2.2.2]octane
- salts of 1-[2-({4-[2-(1H-benzotriazol-6-yl)diazenyl]-3-methylphenyl}ethylamino)ethyl]-4-aza-1-azoniabicyclo[2.2.2]octane
- salts of 1-[2-({4-[2-(1H-benzotriazol-6-yl)diazenyl]-3-chlorophenyl}ethylamino)ethyl]-4-aza-1-azoniabicyclo[2.2.2]octane.

4. The agent for dyeing and optionally simultaneously lightening human hair according to one of claims 1 to 3, **characterized in that** it contains the compound(s) of formula (I) in amounts of from 0.001 to 5 wt.%, preferably from 0.0025 to 2.5 wt.%, particularly preferably from 0.005 to 1.5 wt.%, and in particular from 0.01 to 1 wt.%, in each case based on the total agent.

5. The agent for dyeing and optionally simultaneously lightening human hair according to one of claims 1 to 4, **characterized in that** it additionally contains, based on the weight thereof, from 0.001 to 5 wt.% of one or more oxidation dye precursors and/or direct dyes.

6. The agent for dyeing and optionally simultaneously lightening human hair according to one of claims 1 to 5, **characterized in that** it contains from 0.5 to 15 wt.%, preferably from 1 to 12.5 wt.%, particularly preferably from 2.5 to 10 wt.%, and in particular from 3 to 6 wt.%, of hydrogen peroxide (calculated as 100% H₂O₂).

7. A method for dyeing and optionally simultaneously lightening human hair, **characterized in that**
- a pre-treatment agent M1 is applied to the fiber if desired, then
- an agent M2 is used on the fiber, an additional agent M3 being added, if desired, to the agent M2 before said agent is used,
- said agent M2 is rinsed off the fiber after a period of 5-30 minutes,
- and a post-treatment agent M4 is optionally applied to the fiber after treatment and is rinsed off again after a contact time of several minutes,
at least one of the agents M1, M2 or M3 being an agent according to one of claims 1 to 4.

8. The use of cationic compounds of formula (I): 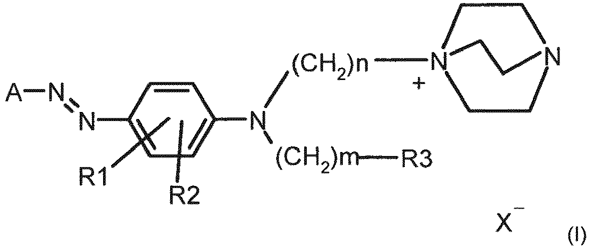 in which
A is a heterocyclic or carbocyclic, optionally substituted, uncharged ring system,
R¹, R², R³ represent, independently of one another, a hydrogen atom, a C₁-C₆ alkyl group, a C₂-C₆ alkenyl group, a C₁-C₆ alkoxy group, a halogen atom (CI, Br, J or F), a hydroxy group, an amino group, a carboxyl group, a C₁-C₆ alkylamino group, a C₁-C₆ dialkylamino group, a nitro group, a nitrile group, a hydroxy-C₁-C₆ alkyloxy group or an acylamino group,
n, m represent, independently of one another, an integer from 1 to 6,
X⁻ represents a monovalent anion, preferably halide, hydrogen sulfate, ½ sulfate, p-benzene sulfonate, p-toluene sulfonate, acetate, tetrafluoroborate, trifluoromethane sulfonate, hexafluorophosphate, methane sulfate or methane sulfonate in agents for dyeing and optionally simultaneously lightening human hair in order to obtain color shades that have very good fastness properties.

9. Cationic compounds of formula (I) according to claim 1 selected from:
- salts of 1-[2-({4-[2-(4,5-dicyanoimidazol-2-yl)diazenyl]phenyl}ethylamino)ethyl]-4-aza-1-azoniabicyclo[2.2.2]octane
- salts of 1-[2-({4-[2-(4,5-dicyanoimidazol-2-yl)diazenyl]-3-methylphenyl}ethylamino)ethyl]-4-aza-1-azoniabicyclo[2.2.2]octane
- salts of 1-[2-({4-[2-(4,5-dicyanoimidazol-2-yl)diazenyl]-3-chlorophenyl}ethylamino)ethyl]-4-aza-1-azoniabicyclo[2.2.2]octane
- salts of 1-[2-({4-[2-(1,2,4-4H-triazol-3-yl)diazenyl)phenyl}ethylamino)ethyl]-4-aza-1-azoniabicyclo[2.2.2]octane
- salts of 1-[2-({4-[2-(1,2,4-4H-triazol-3-yl)diazenyl]-3-methylphenyl}ethylamino)ethyl]-4-aza-1-azoniabicyclo[2.2.2]octane
- salts of 1-[2-({4-[2-(1,2,4-4H-triazol-3-yl)diazenyl]-3-chlorophenyl}ethylamino)ethyl]-4-aza-1-azoniabicyclo-[2.2.2]octane
- salts of 1-[2-({4-[2-(5-phenyl-1H-pyrazol-3-yl)diazenyl]phenyl}ethylamino)ethyl]-4-aza-1-azoniabicyclo[2.2.2]octane
- salts of 1-[2-({4-[2-(5-phenyl-1H-pyrazol-3-yl)diazenyl]-3-methylphenyl}ethylamino)ethyl]-4-aza-1-azoniabicyclo[2.2.2]octane
- salts of 1-[2-({4-[2-(5-phenyl-1H-pyrazol-3-yl)diazenyl]-3-chlorophenyl}ethylamino)ethyl]-4-aza-1-azoniabicyclo-[2.2.2]octane
- salts of 1-[2-({4-[2-(thiazol-2-yl)diazenyl]phenyl}ethylamino)ethyl]-4-aza-1-azoniabicyclo[2.2.2]octane
- salts of 1-[2-({4-[2-(thiazol-2-yl)diazenyl]-3-methylphenyl}ethylamino)ethyl]-4-aza-1-azoniabicyclo[2.2.2]octane
- salts of 1-[2-({4-[2-(thiazol-2-yl)diazenyl]-3-chlorophenyl}ethylamino)ethyl]-4-aza-1-azoniabicyclo-[2.2.2]octane
- salts of 1-[2-({4-[2-(5-nitrothiazol-2-yl)diazenyl]phenyl}ethylamino)ethyl]-4-aza-1-azoniabicyclo[2.2.2]octane
- salts of 1-[2-({4-[2-(5-nitrothiazol-2-yl)diazenyl]-3-methylphenyl}ethylamino)ethyl]-4-aza-1-azoniabicyclo[2.2.2]octane
- salts of 1-[2-({4-[2-(5-nitrothiazol-2-yl)diazenyl]-3-chlorophenyl}ethylamino)ethyl]-4-aza-1-azoniabicyclo-[2.2.2]octane
- salts of 1-[2-({4-[2-(3-phenyl-1,2,4-thiadiazol-5-yl)diazenyl]phenyl}ethylamino)ethyl]-4-aza-1-azoniabicyclo[2.2.2]octane
- salts of 1-[2-({4-[2-(3-phenyl-1,2,4-thiadiazol-5-yl)diazenyl]-3-methylphenyl)ethylamino)ethyl]-4-aza-1-azoniabicyclo[2.2.2]octane
- salts of 1-[2-({4-[2-(3-phenyl-1,2,4-thiadiazol-5-yl)diazenyl]-3-chlorophenyl}ethylamino)ethyl]-4-aza-1-azoniabicyclo-[2.2.2]octane
- salts of 1-[2-({4-[2-(5-nitro-1H-benzimidazol-2-yl)diazenyl]phenyl}ethylamino)ethyl]-4-aza-1-azoniabicyclo[2.2.2]octane
- salts of 1-[2-({4-[2-(5-nitro-1H-benzimidazol-2-yl)diazenyl]-3-methylphenyl}ethylamino)ethyl]-4-aza-1-azoniabicyclo[2.2.2]octane
- salts of 1[2-({4-[2-(5-nitro-1H-benzimidazol-2-yl)diazenyl]-3-chlorophenyl}ethylamino)ethyl]-4-aza-1-azoniabicyclo-[2.2.2]octane
- salts of 1-[2-({4-[2-(5-nitro-benzothiazol-2-yl)diazenyl]phenyl}ethylamino)ethyl]-4-aza-1-azoniabicyclo[2.2.2]octane
- salts of 1-[2-({4-[2-(5-nitro-benzothiazol-2-yl)diazenyl]-3-methylphenyl}ethylamino)ethyl]-4-aza-1-azoniabicyclo[2.2.2]octane
- salts of 1-[2-({4-[2-(5-nitro-benzothiazol-2-yl)diazenyl]-3-chlorophenyl}ethylamino)ethyl]-4-aza-1-azoniabicyclo-[2.2.2]octane
- salts of 1-{2-[(4-{2-[6-(methylsulfonyl)benzothiazol-2-yl]diazeny}phenyl)-ethylamino]ethyl}-4-aza-1-azoniabicyclo[2.2.2]octane
- salts of 1-{2-[(4-{2-[6-(methylsulfonyl)benzothiazol-2-yl]diazeny}-3-methylphenyl)-ethylamino]ethyl}-4-aza-1-azoniabicyclo[2.2.2]octane
- salts of 1-{2-[(4-{2-[6-(methylsulfonyl)benzothiazol-2-yl]diazeny}-3-chlorophenyl)-ethylamino]ethyl}-4-aza-1-azoniabicyclo[2.2.2]octane
- salts of 1-[2-({4-[2-(5-methoxybenzothiazol-2-yl)diazenyl]phenyl}ethylamino)ethyl]-4-aza-1-azoniabicyclo[2.2.2]octane
- salts of 1-[2-({4-[2-(5-methoxybenzothiazol-2-yl)diazenyl]-3-methylphenyl}ethylamino)ethyl]-4-aza-1-azoniabicyclo[2.2.2]octane
- salts of 1-[2-({4-[2-(5-methoxybenzothiazol-2-yl)diazenyl]-3-chlorophenyl}ethylamino)ethyl]-4-aza-1-azoniabicyclo-[2.2.2]octane
- salts of 1-[2-({4-[2-(1H-benzotriazol-6-yl)diazenyl]phenyl}ethylamino)ethyl]-4-aza-1-azoniabicyclo[2.2.2]octane
- salts of 1-[2-({4-[2-(1H-benzotriazol-6-yl)diazenyl]-3-methylphenyl}ethylamino)ethyl]-4-aza-1-azoniabicyclo[2.2.2]octane
- salts of 1-[2-({4-[2-(1H-benzotriazol-6-yl)diazenyl]-3-chlorophenyl}ethylamino)ethyl]-4-aza-1-azoniabicyclo[2.2.2]octane

## Revendications

1. Agent de coloration, et le cas échéant d'éclaircissement simultané de cheveux humains, contenant au moins un composé cationique de la formule (I) : 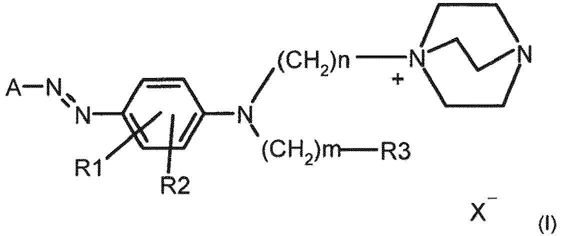 dans laquelle
A représente un cycle benzène le cas échéant substitué ou un cycle naphtalène le cas échéant substitué des formules générales (II) ou (III) : 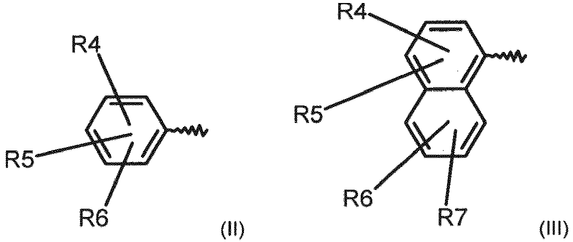
dans lesquelles
R⁴, R⁵, R⁶ et R⁷ représentent indépendamment l'un de l'autre un atome d'hydrogène, un groupe nitro, un groupe d'acide sulfonique, un groupe nitrile, un atome d'halogène (chlorure, bromure ou fluorure), un groupe carbamoyle, un groupe alkylesulfonyle en C₁-C₆, un groupe sulfamoyle ou un groupe alcoxy en C₁-C₆, ou
A représente un des hétérocycles (IV) à (XV) présentés ci-dessous 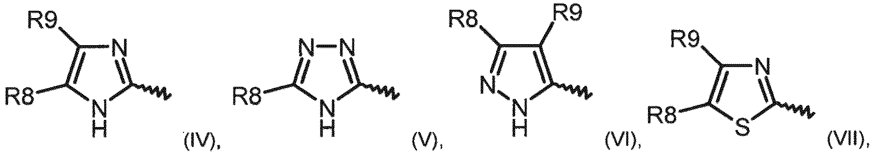 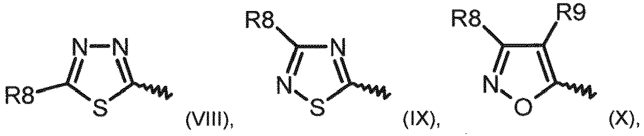 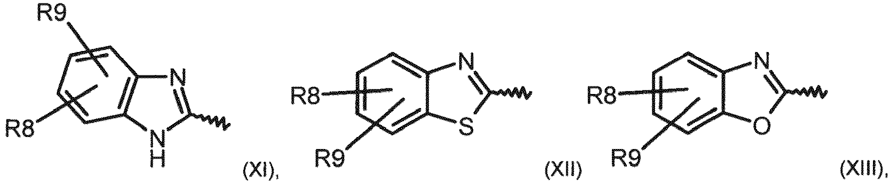 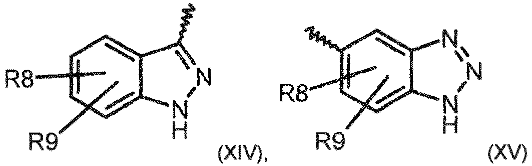
dans lesquels
R⁸, R⁹ représentent indépendamment l'un de l'autre un atome d'hydrogène, un groupe nitro, un atome d'halogène, un groupe alkylesulfonyle en C₁-C₆, un groupe carbamoyle, un groupe alkyle en C₁-C₆, un groupe alcoxy en C₁-C₆ ou un groupe phényle,
R¹, R², R³ représentent indépendamment l'un de l'autre un atome d'hydrogène, un groupe alkyle en C₁-C₆, un groupe alkényle en C₂-C₆, un groupe alcoxy en C₁-C₆, un atome d'halogène (Cl, Br, J ou F), un groupe hydroxy, un groupe amino, un groupe carboxyle, un groupe alkylamino en C₁-C₆, un groupe dialkylamino en C₁-C₆, un groupe nitro, un groupe nitrile, un groupe alkyloxy hydroxy en C₁-C₆ ou un groupe acylamino,
n, m représentent indépendamment l'un de l'autre un nombre entier allant de 1 - 6,
X⁻ représente un anion monovalent, de préférence de l'halogénure, du sulfate d'hydrogène, du ½ sulfate, du p-benzènesulfonate, du p-toluolsulfonate, de l'acétate, du tétrafluoroborate, du trifluorméthanesulfonate, de l'hexafluorophosphate, du méthanesulfate ou du méthanesulfonate.

2. Agent de coloration et le cas échéant d'éclaircissement simultané de cheveux humains, selon la revendication 1, **caractérisé en ce qu'**il contient au moins un composé cationique choisi parmi le groupe constitué par
- les sels du 1-[2-({4-[2-(4-nitrophényl)diazényl]phényl}éthylamino)éthyl]-4-aza-1-azoniabicyclo[2.2.2]octane
- les sels du 1-[2-({4-[2-(4-nitrophényl)diazényl]-3-méthylphényl}éthylamino)éthyl]-4-aza-1-azoniabicyclo[2.2.2]octane
- les sels du 1-[2-({4-[2-(4-nitrophényl)diazényl]-3-chlorophényl}éthylamino)éthyl]-4-aza-1-azoniabicyclo[2.2.2]octane
- les sels du 1-[2-({4-[2-(2-cyano-4-nitrophényl)diazényl]phényl}éthylamino)éthyl]-4-aza-1-azoniabicyclo[2.2.2]octane
- les sels du 1-[2-({4-[2-(2-cyano-4-nitrophényl)diazényl]-3-méthylphényl}éthylamino)éthyl]-4-aza-1-azoniabicyclo[2.2.2]octane
- les sels du 1-[2-({4-[2-(2-cyano-4-nitrophényl)diazényl]-3-chlorophényl}éthylamino)éthyl]-4-aza-1-azoniabicyclo[2.2.2]octane
- les sels du 1-[2-({4-[2-(2-chloro-4-nitrophényl)diazényl]phényl}éthylamino)éthyl]-4-aza-1-azoniabicyclo[2.2.2]octane
- les sels du 1-[2-({4-[2-(2-chloro-4-nitrophényl)diazényl]-3-méthylphényl}éthylamino)éthyl]-4-aza-1-azoniabicyclo[2.2.2]octane
- les sels du 1-[2-({4-[2-(2-chloro-4-nitrophényl)diazényl]-3-chlorophényl}éthylamino)éthyl]-4-aza-1-azoniabicyclo[2.2.2]octane
- les sels du 1-[2-({4-[2-(2-chloro-6-cyano-4-nitrophényl)diazényl]phényl}éthylamino)éthyl]-4-aza-1-azoniabicyclo[2.2.2]octane
- les sels du 1-[2-({4-[2-(2-chloro-6-cyano-4-nitrophényl)diazényl]-3-méthylphényl}éthylamino)éthyl]-4-aza-1-azoniabicyclo[2.2.2]octane
- les sels du 1-[2-({4-[2-(2-chloro-6-cyano-4-nitrophényl)diazényl]-3-chlorophényl}éthylamino)éthyl]-4-aza-1-azoniabicyclo[2.2.2]octane
- les sels du 1-[2-({4-[2-(2,6-dichloro-4-nitrophényl)-diazényl]phényl}éthylamino)éthyl]-4-aza-1-azoniabicyclo[2.2.2]octane
- les sels du 1-[2-({4-[2-(2,6-dichloro-4-nitrophényl)diazényl]-3-méthylphényl}éthylamino)éthyl]-4-aza-1-azoniabicyclo[2.2.2]octane
- les sels du 1-[2-({4-[2-(2,6-dichloro-4-nitrophényl)diazényl]-3-chlorophényl}éthylamino)éthyl]-4-aza-1-azoniabicyclo[2.2.2]octane
- les sels du 1-[2-({4-[2-(4-méthoxy-2-nitrophényl)diazényl]phényl}éthylamino)éthyl]-4-aza-1-azoniabicyclo[2.2.2]octane
- les sels du 1-[2-({4-[2-(4-méthoxy-2-nitrophényl)diazényl]-3-méthylphényl}éthylamino)éthyl]-4-aza-1-azoniabicyclo[2.2.2]octane
- les sels du 1-[2-({4-[2-(4-méthoxy-2-nitrophényl)diazényl]-3-chlorophényl}éthylamino)éthyl]-4-aza-1-azoniabicyclo[2.2.2]octane
- les sels du 1-[2-({4-[2-(2-méthoxy-4-nitrophényl)diazényl]phényl}éthylamino)éthyl]-4-aza-1-azoniabicyclo[2.2.2]octane
- les sels du 1-[2-({4-[2-(2-méthoxy-4-nitrophényl)diazényl]-3-méthylphényl}éthylamino)éthyl]-4-aza-1-azoniabicyclo[2.2.2]octane
- les sels du 1-[2-({4-[2-(2-méthoxy-4-nitrophényl)diazényl]-3-chlorophényl}éthylamino)éthyl]-4-aza-1-azoniabicyclo[2.2.2]octane
- les sels du 1-[2-({4-[2-(2-méthyl-4-nitrophényl)diazényl]phényl}éthylamino)éthyl]-4-aza-1-azoniabicyclo[2.2.2]octane
- les sels du 1-[2-({4-[2-(2-méthyl-4-nitrophényl)diazényl]-3-méthylphényl}éthylamino)éthyl]-4-aza-1-azoniabicyclo[2.2.2]octane
- les sels du 1-[2-({4-[2-(2-méthyl-4-nitrophényl)diazényl]-3-chlorophényl}éthylamino)éthyl]-4-aza-1-azoniabicyclo[2.2.2]octane.

3. Agent de coloration et le cas échéant d'éclaircissement simultané de cheveux humains selon l'une des revendications 1 à 2, **caractérisé en ce qu'**il contient au moins un composé cationique choisi parmi le groupe constitué par
- les sels du 1-[2-({4-[2-(4,5-dicyanoimidazol-2-yl)diazényl]phényl}éthylamino)éthyl]-4-aza-1-azoniabicyclo[2.2.2]octane
- les sels du 1-[2-({4-[2-(4,5-dicyanoimidazol-2-yl)diazényl]-3-méthylphényl}éthylamino)éthyl]-4-aza-1-azoniabicyclo[2.2.2]octane
- les sels du 1-[2-({4-[2-(4,5-dicyanoimidazol-2-yl)diazényl]-3-chlorophényl}éthylamino)éthyl]-4-aza-1-azoniabicyclo[2.2.2]octane
- les sels du 1-[2-({4-[2-(1,2,4-4H-triazol-3-yl)diazényl]phényl}éthylamino)éthyl]-4-aza-1-azoniabicyclo[2.2.2]octane
- les sels du 1-[2-({4-[2-(1,2,4-4H-triazol-3-yl)diazényl]-3-méthylphényl}éthylamino)éthyl]-4-aza-1-azoniabicyclo[2.2.2]octane
- les sels du 1-[2-({4-[2-(1,2,4-4H-triazol-3-yl)diazényl]-3-chlorophényl}éthylamino)éthyl]-4-aza-1-azoniabicyclo[2.2.2]octane
- les sels du 1-[2-({4-[2-(5-phényl-1H-pyrazol-3-yl)diazényl]phényl}éthylamino)éthyl]-4-aza-1-azoniabicyclo[2.2.2]octane
- les sels du 1-[2-({4-[2-(5-phényl-1H-pyrazol-3-yl)diazényl]-3-méthylphényl}éthylamino)éthyl]-4-aza-1-azoniabicyclo[2.2.2]octane
- les sels du 1-[2-({4-[2-(5-phényl-1H-pyrazol-3-yl)diazényl]-3-chlorophényl}éthylamino)éthyl]-4-aza-1-azoniabicyclo[2.2.2]octane
- les sels du 1-[2-({4-[2-(thiazol-2-yl)diazényl]phényl}éthylamino)éthyl]-4-aza-1-azoniabicyclo[2.2.2]octane
- les sels du 1-[2-({4-[2-(thiazol-2-yl)diazényl]-3-méthylphényl}éthylamino)éthyl]-4-aza-1-azoniabicyclo[2.2.2]octane
- les sels du 1-[2-({4-[2-(thiazol-2-yl)diazényl]-3-chlorophényl}éthylamino)éthyl]-4-aza-1-azoniabicyclo[2.2.2]octane
- les sels du 1-[2-({4-[2-(5-nitrothiazol-2-yl)diazényl]phényl}éthylamino)éthyl]-4-aza-1-azoniabicyclo[2.2.2]octane
- les sels du 1-[2-({4-[2-(5-nitrothiazol-2-yl)diazényl]-3-méthylphényl}éthylamino)éthyl]-4-aza-1-azoniabicyclo[2.2.2]octane
- les sels du 1-[2-({4-[2-(5-nitrothiazol-2-yl)diazényl]-3-chlorophényl}éthylamino)éthyl]-4-aza-1-azoniabicyclo[2.2.2]octane
- les sels du 1-[2-({4-[2-(3-phényl-1,2,4-thiadiazol-5-yl)diazényl]phényl}éthylamino)éthyl]-4-aza-1-azoniabicyclo[2.2.2]octane
- les sels du 1-[2-({4-[2-(3-phényl-1,2,4-thiadiazol-5-yl)diazényl]-3-méthylphényl}éthylamino)éthyl]-4-aza-1-azoniabicyclo[2.2.2]octane
- les sels du 1-[2-({4-[2-(3-phényl-1,2,4-thiadiazol-5-yl)diazényl]-3-chlorophényl}éthylamino)éthyl]-4-aza-1-azoniabicyclo[2.2.2]octane
- les sels du 1-[2-({4-[2-(5-nitro-1H-benzimidazol-2-yl)diazényl]phényl}éthylamino)éthyl]-4-aza-1-azoniabicyclo[2.2.2]octane
- les sels du 1-[2-({4-[2-(5-nitro-1H-benzimidazol-2-yl)diazényl]-3-méthylphényl}éthylamino)éthyl]-4-aza-1-azoniabicyclo[2.2.2]octane
- les sels du 1-[2-({4-[2-(5-nitro-1H-benzimidazol-2-yl)diazényl]-3-chlorophényl}éthylamino)éthyl]-4-aza-1-azoniabicyclo[2.2.2]octane
- les sels du 1-[2-({4-[2-(5-nitro-benzothiazol-2-yl)diazényl]phényl}éthylamino)éthyl]-4-aza-1-azoniabicyclo[2.2.2]octane
- les sels du 1-[2-({4-[2-(5-nitro-benzothiazol-2-yl)diazényl]-3-méthylphényl}éthylamino)éthyl]-4-aza-1-azoniabicyclo[2.2.2]octane
- les sels du 1-[2-({4-[2-(5-nitro-benzothiazol-2-yl)diazényl]-3-chlorophényl}éthylamino)éthyl]-4-aza-1-azoniabicyclo[2.2.2]octane
- les sels du 1-{2-[(4-{2-[6-(méthylsulfonyle)benzothiazol-2-yl]diazény}phényl)-éthylamino]éthyl}-4-aza-1-azoniabicyclo[2.2.2]octane
- les sels du 1-{2-[(4-{2-[6-(méthylsulfonyle)benzothiazol-2-yl]diazény}-3-méthylphényl)-éthylamino]éthyl}-4-aza-1-azoniabicyclo[2.2.2]octane
- les sels du 1-{2-[(4-{2-[6-(méthylsulfonyle)benzothiazol-2-yl]diazény}-3-chlorophényl)-éthylamino]éthyl}-4-aza-1-azoniabicyclo[2.2.2]octane
- les sels du 1-[2-({4-[2-(5-méthoxybenzothiazol-2-yl)diazényl]phényl}éthylamino)éthyl]-4-aza-1-azoniabicyclo[2.2.2]octane
- les sels du 1-[2-({4-[2-(5-méthoxybenzothiazol-2-yl)diazényl]-3-méthylphényl}éthylamino)éthyl]-4-aza-1-azoniabicyclo[2.2.2]octane
- les sels du 1-[2-({4-[2-(5-méthoxybenzothiazol-2-yl)diazényl]-3-chlorophényl}éthylamino)éthyl]-4-aza-1-azoniabicyclo[2.2.2]octane
- les sels du 1-[2-({4-[2-(1H-benzotriazol-6-yl)diazényl]phényl}éthylamino)éthyl]-4-aza-1-azoniabicyclo[2.2.2]octane
- les sels du 1-[2-({4-[2-(1H-benzotriazol-6-yl)diazényl]-3-méthylphényl}éthylamino)éthyl]-4-aza-1-azoniabicyclo[2.2.2]octane
- les sels du 1-[2-({4-[2-(1H-benzotriazol-6-yl)diazényl]-3-chlorophényl}éthylamino)éthyl]-4-aza-1-azoniabicyclo[2.2.2]octane.

4. Agent de coloration et le cas échéant d'éclaircissement simultané de cheveux humains selon l'une des revendications 1 à 3, **caractérisé en ce qu'**il contient le(s) composé(s) de la formule (I) dans des quantités allant de 0,001 à 5 % en poids, de préférence de 0,0025 à 2,5 % en poids, de manière particulièrement préférée de 0,005 à 1,5 % en poids et en particulier de 0,01 à 5 % en poids, rapporté à l'ensemble de l'agent.

5. Agent de coloration et le cas échéant d'éclaircissement simultané de cheveux humains selon l'une des revendications 1 à 4, **caractérisé en ce qu'**il contient en outre - rapporté à son poids - de 0,001 à 5 % en poids d'un ou plusieurs précurseurs de colorant par oxydation et/ou colorants à action directe.

6. Agent de coloration et le cas échéant d'éclaircissement simultané de cheveux humains selon l'une des revendications 1 à 5, **caractérisé en ce qu'**il contient de 0,5 à 15 % en poids, de préférence de 1 à 12,5 % en poids, de manière particulièrement préférée de 2,5 à 10 % en poids et en particulier de 3 à 6 % en poids de peroxyde d'hydrogène (compté comme contenant 100 % de H₂O₂).

7. Procédé de coloration et le cas échéant d'éclaircissement simultané de cheveux humains, **caractérisé en ce que**
- éventuellement, un agent de pré-traitement M1 est appliqué sur les fibres, puis
- un agent M2 est utilisé sur les fibres, un autre agent M3 étant éventuellement ajouté à l'agent M2 avant utilisation,
- cet agent M2 est rincé des fibres après une durée de 5-30 minutes
- et après le traitement, un agent de post-traitement est éventuellement appliqué sur les fibres puis est de nouveau rincé après un temps d'action de quelques minutes,
au moins un des agents M1, M2 ou M3 étant un agent selon l'une des revendications 1 à 4.

8. Utilisation de composés cationiques de la formule (I) : 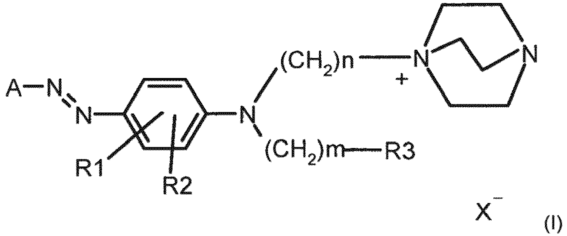 dans laquelle
A est un système cyclique hétérocyclique ou carbocyclique, le cas échéant substitué, non chargé
R¹, R², R³ représentent indépendamment l'un de l'autre un atome d'hydrogène, un groupe alkyle en C₁-C₆, un groupe alkényle en C₂-C₆, un groupe alcoxy en C₁-C₆, un atome d'halogène (Cl, Br, J ou F), un groupe hydroxy, un groupe amino, un groupe carboxyle, un groupe alkylamino en C₁-C₆, un groupe dialkylamino en C₁-C₆, un groupe nitro, un groupe nitrile, un groupe alkyloxy hydroxy en C₁-C₆ ou un groupe acylamino,
n, m représentent indépendamment l'un de l'autre un nombre entier allant de 1 - 6,
X⁻ représente un anion monovalent, de préférence de l'halogénure, du sulfate d'hydrogène, du ½ sulfate, du p-benzènesulfonate, du p-toluolsulfonate, de l'acétate, du tétrafluoroborate, du trifluorméthanesulfonate, de l'hexafluorophosphate, du méthanesulfate ou du méthanesulfonate
dans des agents de coloration et le cas échéant d'éclaircissement simultané de cheveux humains, afin d'obtenir des nuances de couleur présentant de très bonnes propriétés d'authenticité.

9. Composés cationiques de la formule (I) selon la revendication 1, choisis parmi :
- les sels du 1-[2-({4-[2-(4,5-dicyanoimidazol-2-yl)diazényl]phényl}éthylamino)éthyl]-4-aza-1-azoniabicyclo[2.2.2]octane
- les sels du 1-[2-({4-[2-(4,5-dicyanoimidazol-2-yl)diazényl]-3-méthylphényl}éthylamino)éthyl]-4-aza-1-azoniabicyclo[2.2.2]octane
- les sels du 1-[2-({4-[2-(4,5-dicyanoimidazol-2-yl)diazényl]-3-chlorophényl}éthylamino)éthyl]-4-aza-1-azoniabicyclo[2.2.2]octane
- les sels du 1-[2-({4-[2-(1,2,4-4H-triazol-3-yl)diazényl]phényl}éthylamino)éthyl]-4-aza-1-azoniabicyclo[2.2.2]octane
- les sels du 1-[2-({4-[2-(1,2,4-4H-triazol-3-yl)diazényl]-3-méthylphényl}éthylamino)éthyl]-4-aza-1-azoniabicyclo[2.2.2]octane
- les sels du 1-[2-({4-[2-(1,2,4-4H-triazol-3-yl)diazényl]-3-chlorophényl}éthylamino)éthyl]-4-aza-1-azoniabicyclo[2.2.2]octane
- les sels du 1-[2-({4-[2-(5-phényl-1H-pyrazol-3-yl)diazényl]phényl}éthylamino)éthyl]-4-aza-1-azoniabicyclo[2.2.2]octane
- les sels du 1-[2-({4-[2-(5-phényl-1H-pyrazol-3-yl)diazényl]-3-méthylphényl}éthylamino)éthyl]-4-aza-1-azoniabicyclo[2.2.2]octane
- les sels du 1-[2-({4-[2-(5-phényl-1H-pyrazol-3-yl)diazényl]-3-chlorophényl}éthylamino)éthyl]-4-aza-1-azoniabicyclo[2.2.2]octane
- les sels du 1-[2-({4-[2-(thiazol-2-yl)diazényl]phényl}éthylamino)éthyl]-4-aza-1-azoniabicyclo[2.2.2]octane
- les sels du 1-[2-({4-[2-(thiazol-2-yl)diazényl]-3-méthylphényl}éthylamino)éthyl]-4-aza-1-azoniabicyclo[2.2.2]octane
- les sels du 1-[2-({4-[2-(thiazol-2-yl)diazényl]-3-chlorophényl}éthylamino)éthyl]-4-aza-1-azoniabicyclo[2.2.2]octane
- les sels du 1-[2-({4-[2-(5-nitrothiazol-2-yl)diazényl]phényl}éthylamino)éthyl]-4-aza-1-azoniabicyclo[2.2.2]octane
- les sels du 1-[2-({4-[2-(5-nitrothiazol-2-yl)diazényl]-3-méthylphényl}éthylamino)éthyl]-4-aza-1-azoniabicyclo[2.2.2]octane
- les sels du 1-[2-({4-[2-(5-nitrothiazol-2-yl)diazényl]-3-chlorophényl}éthylamino)éthyl]-4-aza-1-azoniabicyclo[2.2.2]octane
- les sels du 1-[2-({4-[2-(3-phényl-1,2,4-thiadiazol-5-yl)diazényl]phényl}éthylamino)éthyl]-4-aza-1-azoniabicyclo[2.2.2]octane
- les sels du 1-[2-({4-[2-(3-phényl-1,2,4-thiadiazol-5-yl)diazényl]-3-méthylphényl}éthylamino)éthyl]-4-aza-1-azoniabicyclo[2.2.2]octane
- les sels du 1-[2-({4-[2-(3-phényl-1,2,4-thiadiazol-5-yl)diazényl]-3-chlorophényl}éthylamino)éthyl]-4-aza-1-azoniabicyclo[2.2.2]octane
- les sels du 1-[2-({4-[2-(5-nitro-1H-benzimidazol-2-yl)diazényl]phényl}éthylamino)éthyl]-4-aza-1-azoniabicyclo[2.2.2]octane
- les sels du 1-[2-({4-[2-(5-nitro-1H-benzimidazol-2-yl)diazényl]-3-méthylphényl}éthylamino)éthyl]-4-aza-1-azoniabicyclo[2.2.2]octane
- les sels du 1-[2-({4-[2-(5-nitro-1H-benzimidazol-2-yl)diazényl]-3-chlorophényl}éthylamino)éthyl]-4-aza-1-azoniabicyclo[2.2.2]octane
- les sels du 1-[2-({4-[2-(5-nitro-benzothiazol-2-yl)diazényl]phényl}éthylamino)éthyl]-4-aza-1-azoniabicyclo[2.2.2]octane
- les sels du 1-[2-({4-[2-(5-nitro-benzothiazol-2-yl)diazényl]-3-méthylphényl}éthylamino)éthyl]-4-aza-1-azoniabicyclo[2.2.2]octane
- les sels du 1-[2-({4-[2-(5-nitro-benzothiazol-2-yl)diazényl]-3-chlorophényl}éthylamino)éthyl]-4-aza-1-azoniabicyclo[2.2.2]octane
- les sels du 1-{2-[(4-{2-[6-(méthylsulfonyl)benzothiazol-2-yl]diazény}phényl)-éthylamino]éthyl}-4-aza-1-azoniabicyclo[2.2.2]octane
- les sels du 1-{2-[(4-{2-[6-(méthylsulfonyl)benzothiazol-2-yl]diazény}-3-méthylphényl)-éthylamino]éthyl}-4-aza-1-azoniabicyclo[2.2.2]octane
- les sels du 1-{2-[(4-{2-[6-(méthylsulfonyl)benzothiazol-2-yl]diazény}-3-chlorophényl)-éthylamino]éthyl}-4-aza-1-azoniabicyclo[2.2.2]octane
- les sels du 1-[2-({4-[2-(5-méthoxybenzothiazol-2-yl)diazényl]phényl}éthylamino)éthyl]-4-aza-1-azoniabicyclo[2.2.2]octane
- les sels du 1-[2-({4-[2-(5-méthoxybenzothiazol-2-yl)diazényl]-3-méthylphényl}éthylamino)éthyl]-4-aza-1-azoniabicyclo[2.2.2]octane
- les sels du 1-[2-({4-[2-(5-méthoxybenzothiazol-2-yl)diazényl]-3-chlorophényl}éthylamino)éthyl]-4-aza-1-azoniabicyclo[2.2.2]octane
- les sels du 1-[2-({4-[2-(1H-benzotriazol-6-yl)diazényl]phényl}éthylamino)éthyl]-4-aza-1-azoniabicyclo[2.2.2]octane
- les sels du 1-[2-({4-[2-(1H-benzotriazol-6-yl)diazényl]-3-méthylphényl}éthylamino)éthyl]-4-aza-1-azoniabicyclo[2.2.2]octane
- les sels du 1-[2-({4-[2-(1H-benzotriazol-6-yl)diazényl]-3-chlorophényl}éthylamino)éthyl]-4-aza-1-azoniabicyclo[2.2.2]octane.
